# EUROPÄISCHE PATENTSCHRIFT

(11) **EP 1 043 399 B1**
(45) Veröffentlichungstag und Bekanntmachung des Hinweises auf die Patenterteilung: **09.04.2003**
(21) Anmeldenummer: 00105929.4
(22) Anmeldetag: 23.03.2000
(51) Int. Cl.: C12N 15/86, C12N 7/01, C12N 7/04, C12N 5/10, C07K 14/18, A61K 49/00, A61K 48/00

(54) **Hepatitis C Virus Zellkultursystem**
Hepatitis C Virus cell culture system
Système de culture du virus de l'hépatite C

(30) Priorität: 03.04.1999 DE 19915178
(43) Veröffentlichungstag der Anmeldung: 11.10.2000
(73) Patentinhaber: Bartenschlager, Ralf, Dr., 55239 Gau-Odernheim (DE)
(72) Erfinder: Bartenschlager, Ralf, Dr., 55239 Gau-Odernheim (DE)
(74) Vertreter: Rudolph, Ulrike, Dr.

(56) Entgegenhaltungen:
- WO-A-98/39031
- WO-A-99/04008
- WO-A-99/67394
- US-A- 5 851 758
- US-A- 5 874 565
- YOO ET AL: "Transfection of differentiated Huh7 with in vitro transcribed HCV RNA and establishment of a culture infected with HCV" JOURNAL OF VIROLOGY, THE AMERICAN SOCIETY FOR MICROBIOLOGY, US, Bd. 69, Nr. 1, 1995, Seiten 32-38, XP002022696 ISSN: 0022-538X
- DASH SRIKANTA ET AL: "Transfection of HepG2 cells with infectious hepatitis C virus genome." AMERICAN JOURNAL OF PATHOLOGY, Bd. 151, Nr. 2, 1997, Seiten 363-373, XP001029512 ISSN: 0002-9440
- DATABASE EMBL [Online] 11. März 1998 (1998-03-11) retrieved from EBI Database accession no. D89815 XP002180379
- DATABASE EMBL [Online] 18. April 1997 (1997-04-18) retrieved from EBI Database accession no. D85516 XP002180641
- DATABASE EMBL [Online] 29. November 1995 (1995-11-29) retrieved from EBI Database accession no. D63922 XP002180380
- LOHMANN V ET AL: "REPLICATION OF SUBGENOMIC HEPATITIS C VIRUS RNAS IN A HEPATOMA CELLLINE" SCIENCE, AMERICAN ASSOCIATION FOR THE ADVANCEMENT OF SCIENCE,, US, Bd. 285, 2. Juli 1999 (1999-07-02), Seiten 110-113, XP000960693 ISSN: 0036-8075
- DATABASE EMBL [Online] 5. Juli 1999 (1999-07-05) retrieved from EBI Database accession no. AJ238799 XP002180381
- BLIGHT K.J. ET AL: 'Efficient initiation of HCV RNA Replication in cell culture' SCIENCE Bd. 290, 08 Dezember 2000, Seiten 1972 - 1974

## Beschreibung

Die Erfindung betrifft ein Hepatitis-C-Virus (HCV)-RNA-Konstrukt mit der Fähigkeit zur Replikation in eukaryontischen Zellen sowie ein Hepatitis C Virus (HCV) Zellkultursystem, das im wesentlichen eukaryontische Zellen umfaßt, die eingeschleustes HCV-spezifisches Genmaterial enthalten, d.h. die mit HCV-spezifischem Genmaterial transfiziert sind.

Das Hepatitis C Virus (HCV) ist eine der Hauptursachen chronischer und sporadischer Leberkrankungen weltweit. Die meisten HCV-Infektionen verlaufen ohne erkennbare klinische Symptome, allerdings werden 80-90% der Infizierten dauerhafte Virusträger und bei 50% dieser dauerhaften Virusträger kommt es zu einer chronischen Leberentzündung mit unterschiedlichen Schweregraden. Ca. 20% der chronisch Infizierten entwickeln im Laufe von 10 bis 20 Jahren eine Leberzirrhose, auf deren Basis sich ein primäres Leberzellkarzinom entwickeln kann. Die chronische Hepatitis C ist heute die Hauptindikation für eine Lebertransplantation. Eine Kausaltherapie gibt es bisher noch nicht. Die einzige derzeit verfügbare Therapie ist die hochdosierte Verabreichung von Interferon-Alpha oder eine Kombination aus Interferon-Alpha und dem Purin-Nukleosidanalogon Ribavirin. Allerdings sprechen nur ca. 60 % aller Behandelten auf diese Therapie an und bei diesen kommt es in mehr als der Hälfte aller Fälle nach dem Absetzen der Behandlung zu einer erneuten Virämie.
Aufgrund der hohen Prävalenz, gerade auch in den Industrieländern, den schwerwiegenden Folgen chronischer Infektionen und dem Fehlen einer Kausaltherapie ist die Entwicklung einer HCV-spezifischen Chemotherapie ein wesentliches Ziel der pharmazeutischen Forschung und Entwicklung. Hauptproblem hierbei ist bisher das Fehlen eines geeigneten Zellkultursystems, das ein Studium der Virus-Replikation und der Pathogenese in eukaryontischen Zellen ermöglicht.

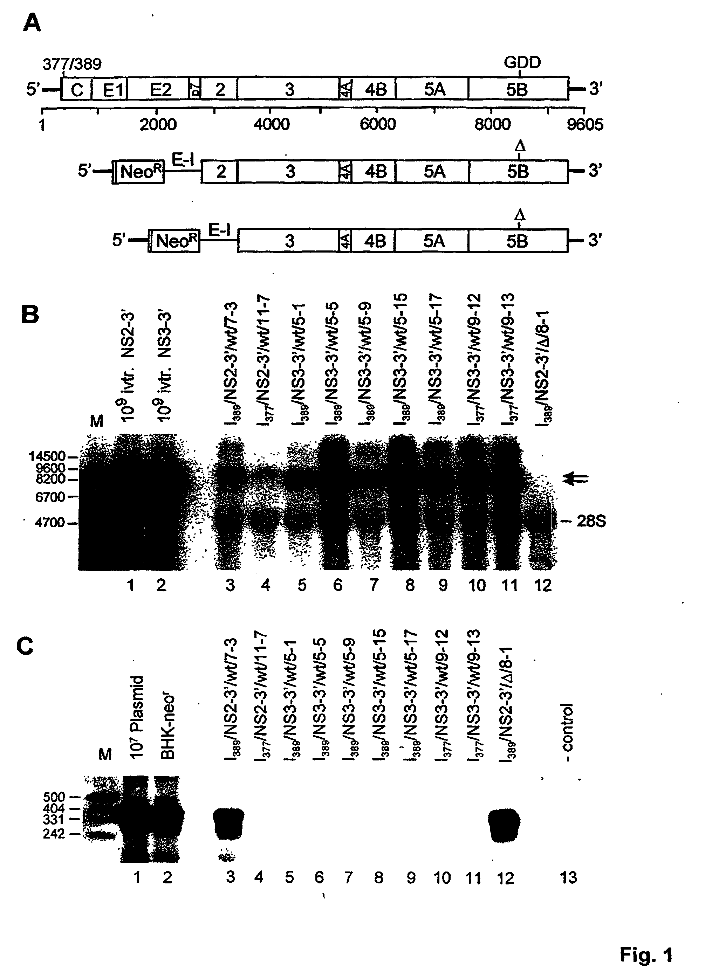

Aufgrund der geringen Virusmengen im Blut bzw. Gewebe, dem Fehlen geeigneter Zellkultursysteme oder Tiermodelle (bis heute ist der Schimpanse das einzige mögliche Versuchstier) sowie dem Fehlen effizienter Systeme zur Produktion virus-ähnlicher Partikel, konnte die molekulare Zusammensetzung des HCV-Partikels bis heute noch nicht eingehend untersucht bzw. aufgeklärt werden. Die derzeit vorliegenden Ergebnisse lassen sich wie folgt zusammenfassen: Das HCV ist ein umhülltes Plusstrang RNA Virus mit einem Partikeldurchmesser von 50-60 nm und einer mittleren Dichte von 1,03-1,1g/ml. Es wurde erstmals 1989 molekular kloniert und charakterisiert (Choo et al., 1989: Science, 244, 359-362). Die HCV-RNA hat eine Länge von ca. 9.6 kb (= 9600 Nukleotide), eine positive Polarität und besitzt ein einziges offenes Leseraster (ORF = open reading frame), das ein lineares Polyprotein von ca 3010 Aminosäuren kodiert (siehe Rice 1996, in Virology, B. N. Fields, D. M. Knipe, P. M. Howley, Eds. (Lippincott-Raven, Philadelphia, PA, 1996), vol. 1, pp.931-960; Clarke 1997, *J. Gen. Virol.* 78, 2397; und Bartenschlager 1997, *Intervirology* 40, 378 und vgl. Fig. 1 A). Bei der Virusreplikation wird das Polyprotein durch zelluläre und virale Proteasen in die reifen und funktionell aktiven Proteine gespalten.
Innerhalb des Polyproteins sind die Proteine wie folgt angeordnet (vom Amino- zum Carboxyterminus): Core-E1-E2-p7-NS2-NS3-NS4A-NS4B-NS5A-NS5B. Das Core-Protein ist die Hauptkomponente des Nukleokapsids. Die Glykoproteine E1 und E2 sind Transmembranproteine und Hauptkomponenten der Virushülle. Sie spielen wahrscheinlich bei der Anheftung des Virus an die Wirtszelle eine wesentliche Rolle. Diese drei Proteine Core, E1 und E2 bauen den Viruspartikel auf und werden deshalb als Strukturproteine bezeichnet. Die Funktion des Proteins p7 ist noch unklar. Das Protein NS2 ist wahrscheinlich die katalytische Domäne der NS2-3 Protease, die für die Prozesierung zwischen den Proteinen NS2 und NS3 verantwortlich ist Das Protein NS3 hat zwei Funktionen, nämlich in der aminoterminalen Domäne eine Proteaseaktivität, die für die Polyproteinprozessierung essentiell ist, und in der carboxyterminalen Domäne eine NTPase/Helikase-Funktion, die wahrscheinlich bei der Replikation der viralen RNA eine Rolle spielt. Das Protein NS4A ist ein Kofaktor der NS3-Protease. Die Funktion des Proteins NS4B ist unbekannt.

Das offene Leseraster ist an seinem 5' Ende von einer ca 340 Nukleotide langen nicht-translatierten Region (NTR = non-translated region) flankiert, die als interne Ribosomenansatzstelle (IRES = internal ribosome entry site) fungiert, und an seinem 3'Ende von einer ca. 230 Nukleotide langen NTR, die höchstwahrscheinlich für die Genomreplikation von Bedeutung ist. Eine solche 3'NTR ist Gegenstand der Patentanmeldung PCT/US 96/14033. Die Strukturproteine in dem amino-terminalen Viertel des Polyproteins werden von der Signalpeptidase der Wirtszelle gespalten. Die Nicht-Strukturproteine (NS) 2 bis (NS) 5B werden von zwei viralen Enzymen prozessiert, nämlich von der NS2-3 und der NS3/4A Proteinase. Die NS3/4A Proteinase wird für alle Spaltungen jenseits des Carboxyterminus von NS3 benötigt. Die Rolle von NS4B ist nicht bekannt. NS5A, ein hoch phosphoryliertes Protein, scheint für die Interferon Resistenz verschiedener HCV-Genotypen verantwortlich zu sein (vgl. Enomoto et al. 1995, *J. Clin. Invest.* 96, 224; Enomoto et al. 1996, *N. Engl. J. Med.* 334, 77; Gale Jr. et al. 1997, *Virology* 230, 217; Kaneko et al. 1994, *Biochem. Biophys. Res. Commun.* 205, 320; Reed et al., 1997, *J. Virol.* 71, 7187) und NS5B wurde als die RNA-abhängige RNA Polymerase identifiziert.

Anhand dieser Erkenntnisse wurden erste Diagnosesysteme entwickelt, die entweder auf dem Nachweis von HCV-spezifischen Antikörpern in Patientenserum oder auf dem Nachweis von HCV-spezifischer RNA mittels RT-PCR (= Reverse Transcription Polymerase Chain Reaction) beruhen, und die mittlerweile routineund/oder vorschriftsmäßig bei allen Blutkonserven angewendet werden (müssen).

Seit der Erstbeschreibung des Genoms 1989 wurden mit Hilfe der PCR-Methode zahlreiche Teil- und Komplettsequenzen des HCV kloniert und charakterisiert. Ein Vergleich dieser Sequenzen zeigt eine hohe Variabilität des viralen Genoms, insbesondere im Bereich des NS5B-Gens, was letztendlich zu einer Einteilung in 6 Genotypen geführt hat, die selbst nochmals in Subtypen a, b, und c untergliedert sind. Die genomische Varianz ist nicht gleichmäßig über das Genom verteilt. So sind die 5'NTR und Teile der 3'NTR hoch konserviert, während bestimmte kodierende Sequenzen z.T. sehr stark variieren, vor allem die Hüllproteine E1 und E2.

Die klonierten und charakterisierten Teil- und Komplettsequenzen des HCV-Genoms wurden außerdem hinsichtlich geeigneter Angriffsziele für ein prospektives antivirales Therapeutikum untersucht. Dabei wurden drei virale Enzyme gefunden, die sich als solches Angriffsziel anbieten. Diese sind (1) der NS3/4A Proteasekomplex, (2) die NS3 Helikase und (3) die NS5B RNA-abhängige RNA Polymerase. Der NS3/4A Proteasekomplex und die NS3 Helikase konnten bereits kristallisiert und hinsichtlich ihrer dreidimensionalen Struktur aufgeklärt werden (Kim et al., 1996, Cell, 87,343; Yem et al., 1998, Protein Science, 7, 837; Love et al., 1996, Cell, 87, 311; Kim et al., 1998, Structure, 6, 89; Yao et al., 1997, Nature Structural Biology, 4, 463, Cho et al., 1998, J. Biol. Chem., 273, 15045); bei der NS5B RNA-abhängigen RNA Polymerase ist dies bis heute noch nicht gelungen.
Obwohl mit diesen Enzymen bedeutsame Angriffsziele für eine Therapieentwicklung der chronischen HCV-Infektion definiert sind, und obwohl sowohl mit Hilfe von 'rational drug design' als auch mit Hilfe von 'high throughput screens' weltweit intensiv nach geeigneten Inhibitoren gesucht wird, leidet die Therapieentwicklung an einem großen Defizit, nämlich dem Fehlen von Zellkultursystemen oder einfachen Tiermodellen, die es erlauben, HCV-RNA oder HCV-Antigene direkt, zuverlässig und mit einfachen laborüblichen Methoden nachzuweisen. Das Fehlen solcher Zellkultursysteme ist auch der Hauptgrund dafür, daß das Verständnis der HCV-Replikation bis heute noch sehr lückenhaft und in weiten Teilen nur hypothetisch ist.

Obwohl nach Meinung der Fachwelt eine enge evolutionäre Beziehung zwischen HCV und den Flavi- und Pestiviren besteht und für diese autonom replizierende RNAs beschrieben sind, die in verschiedenen Zellinien ohne weiteres zur Replikation gebracht werden können und dabei relativ hohe Ausbeuten zeigen ( siehe Khromykh et al., 1997, *J. Virol.* 71, 1497; Behrens et al., 1998, *J. Virol.* 72, 2364; Moser et al., 1998, *J. Virol.* 72, 5318), waren ähnliche Versuche mit HCV bisher nicht erfolgreich.

Zwar ist aus verschiedenen Publikationen bekannt, daß Zellinien oder primäre Zellkulturen mit HCV-haltigem, hochtitrigem Patientenserum infiziert werden können, (Lanford et al. 1994, *Virology* 202, 606; Shimizu et al. 1993, *Procedings of the National Academy of Sciences*, USA, 90, 6037-6041; Mizutani et al. 1996, *Journal of Virology*, 70, 7219-7223; M. Ikeda et al. 1998, *Virus Res.* 56, 157; Fournier et al. 1998, *J. Gen.* *Virol.* 79, 2376 und darin zitierte Literaturstellen, Ito et al. 1996, *Journal of General Virology*, 77, 1043-1054), diese virusinfizierten Zellinien oder Zellkulturen erlauben jedoch nicht den direkten Nachweis von HCV-RNA oder HCV-Antigenen. Die virale RNA in diesen Zellen ist weder in einem Nothern-Blot (einem Standardverfahren zum quantitativen Nachweis von RNA) noch sind die viralen Protein in einem Western-Blot oder mittels Immunpräzipitation detektierbar. Nur mit sehr aufwendigen und indirekten Methoden ist es überhaupt gelungen, eine HCV-Replikation nachzuweisen. Diese nachteiligen Umstände zeigen klar, daß die Replikation in diesen bekannten virusinfizierten Zellinien oder Zellkulturen absolut unzureichend ist.

Desweiteren ist aus den Publikationen von Yoo et al. (1995, *Journal of Virology*, 69, 32-38) und von Dash et al., (1997, *American Journal of Pathology*, 151, 363-373) bekannt, daß Hepatomazellinien mit synthetischer HCV-RNA, die mittels in vitro Trankription von kloniertem HCV-Genom gewonnen wurde, transfiziert werden können. In beiden Publikationen gingen die Autoren von dem Grundgedanken aus, daß das virale HCV-Genom eine Plusstrang-RNA ist, die nach dem Einschleusen in die Zelle direkt als mRNA fungiert, an die sich Ribosomen anheften und im Zuge von Translationsprozessen Virusproteine bilden, aus denen sich letztendlich neue HCV-Partikel bilden (können). Diese Virusreplikation, d.h. diese neu gebildeten HCV-Viren bzw. deren RNA wurde mittels RT-PCR nachgewiesen. Die publizierten Ergebnisse der durchgeführten RT-PCR sprechen jedoch dafür, daß die Effizienz der HCV-Replikation in den beschriebenen HCV-transfizierten Hepatomazellen nur sehr gering ist und jedenfalls nicht ausreicht, um Schwankungen in der Replikationsrate nach gezielter Einwirkung mit prospektiven antiviralen Therapeutika auch nur qualitativ, geschweige denn quantitativ zu messen. Außerdem ist im Stand der Technik mittlerweile bekannt (Yanagi et al., Proc. Natl. Acad. Sci. USA, 96, 2291-95, 1999), daß die hochkonservierte 3' NTR essentiell ist für die Virusreplikation, was in klarem Widerspruch zu den Behauptungen von Yoo et aL und Dash et al. steht, die für ihre Versuche in Unkenntnis des authentischen 3' Endes des HCV-Genoms ausschließlich HCV-Genome mit verkürzten 3' NTRs verwendet haben.

Rice et al. beschreiben in der WO 98 39031 die Herstellung eines infektiösen HCV Genoms und dessen Testung im Schimpansen. Die Autoren behaupten, daß solche infektiösen HCV Genome oder Teilen davon für verschiedene Zwecke, u.a. zum Aufbau von Zellkultursystemen für das HCV geeignet seien, es ist jedoch an keiner Stelle der D1 ein spezifisches, nacharbeitbares HCV-Konstrukt, das tatsächlich in Zellkultur repliziert, beschrieben.

In der WO 99/04008 ist ein kloniertes HCV Genom beschrieben, das im Schimpansen infektiös ist. Eine Infektiosität im Schimpansen erlaubt jedoch nicht den Schluß, daß das betreffende Genom auch in Zellkultur bzw. im In-vitro-Model repliziert.

Die US 5,851,758 beschreibt von der humanen T Zelllinie H9 abgeleitete Zellklone, die mit HCV infizierbar sind, wobei nach der Infektion mit HCV zytopathische Effekte auftreten. Zur Infektion der Zellen wird Patientenserum mit nicht definiertem HCV verwendet. Im Unterschied zum Gegenstand der vorliegenden Patentanmeldung werden keine klonierten HCV-Genome verwendet und insbesondere keine klonierten HCV-Genome mit einem integrierten Selektionsmarker bzw. Reportergen, und die Zellkultur erfolgt nicht mit Hepatomzellen sondern mit humane T-Zellen. Die D5 nimmt die HCV-Konstrukte und das damit hergestellte Zellkulturmodel gemäß geltender Anspruchsfassung deshalb weder vorweg noch legt sie diese/dieses nahe.

In der US 5,874,565 ist eine konservierte Sequenz am 3' Ende des HCV beschrieben, die für die virale Replikation essentiell ist. Es genügt jedoch nicht, die beschriebene 3'-Sequenz an ein beliebiges Genom anzuhängen und dadurch Replikation in Zellkultur zu erhalten, denn diese Sequenz ist zwar notwendig für die Replikation in Zellkultur, aber nicht hinreichend.

Aufgabe der vorliegenden Erfindung ist die Bereitstellung eines Hepatitis-C-Virus (HCV)-RNA-Konstrukts mit der Fähigkeit zur Replikation in eukaryontischen Zellen sowie eines HCV- Zellkultursystems, bei dem die virale RNA in den transfizierten Zellen autonom und mit so hoher Effizienz repliziert, daß Schwankungen in der Replikationsrate nach gezielter Einwirkung mit virus- und insbesondere HCV-spezifischen antiviralen Therapeutika qualitativ und quantitativ und mit Hilfe gängiger, laborüblicher Meßverfahren gemessen werden können.

Eine Lösung dieser Aufgabe besteht in der Bereitstellung eines Hepatitis-C-Virus (HCV)-RNA-Konstrukts der eingangs genannten Art, das sich dadurch auszeichnet, daß es eine Nukleotidsequenz gemäß einem der Sequenzprotokolle SEQ ID NO: 1 bis SEQ ID NO: 11 umfaßt, welche zumindest für die HCV-spezifischen RNA-Abschnitte 5' NTR, NS3, NS4A, NS4B, NS5A, NS5B und 3' NTR und zusätzlich ein selektierbares Markergen (Selektionsgen) kodieren, wobei das selektierbare Markergen das Neomycinphosphotransferasegen ist und wobei anstelle des Neomycinphosphotransferasegens ein anderes Antibiotikaresistenzgen oder sonstiges Resistenzgen enthalten sein kann.
Eine weitere Lösung dieser Aufgabe besteht in der Bereitstellung eines HCV-Zellkultursystems der eingangs genannten Art, bei dem die eukaryontischen Zellen humane Zellen, insbesondere Hepatomazellen sind, die vorzugsweise von einer handelsüblichen Hepatomazellinie abstammen, aber auch aus einer entsprechenden Primärzellkultur gewonnen sein können, und bei dem das eingeschleuste HCV-spezifische Genmaterial ein HCV-RNA-Konstrukt ist, das eine Nukleotidsequenz gemäß einem der Sequenzprotokolle SEQ ID NO: 1 bis SEQ ID NO: 11 umfaßt, welche zumindest für die HCV-spezifischen RNA-Abschnitte 5'NTR, NS3, NS4A, NS4B, NS5A, NS5B und 3' NTR und zusätzlich ein selektierbares Markergen (Selektionsgen) kodieren, wobei das selektierbare Markergen das Neomycinphosphotransferasegen ist und wobei anstelle des Neomycinphosphotransferasegens ein anderes Antibiotikaresistenzgen oder sonstiges Resistenzgen enthalten sein kann. "NTR" steht hier und im folgenden fiir "nicht-translatierte Region" und ist dem einschlägigen Fachmann als Begriff bzw. Abkürzung bekannt und geläufig. Der Begriff "HCV-RNA-Konstrukt" umfaßt hier und im folgenden sowohl Konstrukte, die das komplette HCV-Genom enthalten, als auch solche, die lediglich einen Teil davon, d.h. ein HCV-Subgenom enthalten.

Der Begriff 5' NTR bzw. NS3 bzw. NS4A bzw. NS4B bzw. NS5A bzw. NS5B bzw. 3' NTR umfaßt im vorliegenden Zusammenhang jede Nukleotidsequenz, die im Stand der Technik als Nukleotidsequenz für den jeweils betreffenden funktionellen Abschnitt des HCV-Genoms beschrieben ist.
Die Bereitstellung des erfindungsgemäßen HCV-RNA-Konstrukts ermöglicht erstmals eine detaillierte Analyse der HCV - Replikation, - Pathogenese und - Evolution in Zellkulturen. Die HCV-spezifische virale RNA kann - als vollständiges Genom oder als Subgenom - gezielt in beliebigen Mengen erzeugt werden, und es besteht die Möglichkeit, das RNA-Konstrukt zu manipulieren und damit die HCV-Funktionen auf genetischer Ebene zu untersuchen und aufzuklären.
Da alle zur Zeit als Hauptangriffsziel für eine Therapie untersuchten HCV-Enzyme, nämlich die NS3/4A Protease, die NS3 Helikase und die NS5B Polymerase, in dem erfindungsgemäßen HCV-RNA-Konstrukt enthalten sind, kann es für alle entsprechenden Untersuchungen benutzt werden.

Das in den erfindungsgemäßen HCV-RNA-Konstrukten enthaltene selektierbare Markergen (Selektionsgen), vorzugsweise ein Resistenzgen, insbesondere ein Antibiotikumresistenzgen, hat den Vorteil, daß die mit diesem Konstrukt transfizierten Zellen leicht von den nicht transfizierten Zellen selektiert werden können, indem dem Zellkulturmedium z.B. im Fall eines Antibiotikumresistenzgens das betreffende Antibiotikum zugegeben wird.
Unter 'Antibiotikum' wird im vorliegenden Zusammenhang jede Substanz verstanden, die die nicht-transfizierten Wirtszellen oder die Zellen, in denen die HCV-RNA nur mit geringer Effizienz repliziert, am Leben oder Wachstum hindert, insbesondere Zellgifte wie z.B. Puromycin, Hygromycin, Zeocin, Bleomycin oder Blasticidin.

Eine Alternative zu Antibiotikumresistenzgenen ist z.B. das Thymidin-Kinase-Gen, mit dem eine HAT-Selektion durchgeführt werden kann.

Die Position des selektierbaren Markergens (Selektionsgens), bzw. des bevorzugten Resistenzgens bzw. des besonders bevorzugten Antibiotikumresistenzgens in dem HCV-RNA-Konstrukt liegt vorzugsweise hinter der HCV 5' NTR, d.h. strangabwärts der 5' NTR bzw. strangaufwärts des HCV-Leserasters. Denkbar ist aber auch eine Insertion im Bereich der 3' NTR oder an anderer Stelle des HCV-Genoms oder -Subgenoms, z.B. innerhalb des Polyproteins.

Bei einer alternativen Ausführungsform des erfindungsgemäßen HCV-RNA-Konstrukts ist das selektierbare Markergen (Selektionsgen), insbesondere ein Antibiotikumresistenzgen, über ein Ribozym bzw. eine Erkennungsstelle für ein Ribozym mit der HCV-RNA bzw. der HCV-Genom- oder -Subgenomsequenz verbunden.
Damit geht der Vorteil einher, daß nach erfolgter Selektion derjenigen Zellen, in denen die HCV-RNA produktiv repliziert, in den daraus gewonnenen Zellklonen das Resistenzgen durch ribozymvennittelte Spaltung von der HCV-Subgenomsequenz abgetrennt werden kann, nämlich durch Aktivierung des einklonierten Ribozyms oder, im Fall eines Konstrukts mit einer Erkennungsstelle für ein Ribozym, durch Einschleusen des Ribozyms in die Zellen (z.B. mittels Transfektion eines Ribozymkonstrukts oder Infektion mit einem viralen Expressionsvektor, in den das entsprechende Ribozym eingesetzt wurde). Auf diese Weise wird ein authentisches HCV-Genom-Konstrukt ohne Resistenzgen erhalten, das zur Bildung authentischer infektiöser Viruspartikel befähigt ist.

Eine weitere bevorzugte Ausführungsform des erfindungsgemäßen HCV-RNA-Konstrukts zeichnet sich dadurch aus, daß das Konstrukt anstelle des Markergens oder zusätzlich zu dem Markergen ein integriertes Reportergen aufweist.
Unter Reportergen wird im folgenden jedes Gen verstanden, dessen Anwesenheit sich nach Überführung in einen Zielorganismus leicht und im allgemeinen mit einfachen biochemischen oder auch histochemischen Methoden nachweisen läßt, d.h. das für ein Protein kodiert, welches auch in geringen Mengen einfach und zuverlässig mit den laborüblichen Meßmethoden nachgewiesen und quantifiziert werden kann.
Diese Variante des HCV-RNA-Konstrukts hat den Vorteil, daß der Umfang der Replikation dieses Konstrukts anhand des Reportergenprodukts einfach und schnell mit laborüblichen Methoden gemessen werden kann.
Das Reportergen ist vorzugsweise ein Gen aus der Gruppe bestehend aus den Luziferasegenen, dem CAT-Gen (Chloramphenicol-Acetyl-Transferase-Gen), dem lacZ-Gen (beta-Galaktosidasegen), den GFP-Genen (green-fluorescence-protein-Gen ), dem GUS-Gen (Glukuronidasegen) oder dem SEAP-Gen (Sezemerte-Alkalische-Phosphatase-Gen). Diese Reportergene bzw. deren Produkte, nämlich die entsprechenden Reporterproteine, können z.B. mittels Fluoreszenz, Chemilumineszenz, colorimetrisch oder mit Hilfe immunologischer Methoden (z.B. ELISA) bestimmt werden.
Als Reportergen kommt aber auch ein Surrogatmarkergen in Betracht. Darunter sind in diesem Zusammenhang solche Gene zu verstehen, die für zelluläre Proteine, Nukleinsäuren oder ― allgemein ―für solche Funktionen kodieren, die einer von der Virusreplikation abhängigen Variation unterliegen, und die infolgedessen in denjenigen Zellen, in denen sich das HCV bzw. das HCV-RNA-Konstrukt vermehrt, entweder reprimiert oder aktiviert werden. Das heißt: die Reduktion bzw. Aktivierung dieser Funktion ist ein Ersatzmarker für die Virusreplikation bzw. die Replikation des HCV-RNA-Konstrukts. Eine Variante des erfindungsgemäßen HCV-RNA-Konstrukts ist folglich dadurch gekennzeichnet, daß dessen Replikation die Expression eines (zellulären) Surrogatmarkergens beeinflußt.

Das Reportergen und das selektierbare Markergen können derart räumlich in dem Konstrukt angeordnet sein, daß sie gemeinsam ein Fusionsprotein exprimieren. Hierbei besteht die vorteilhafte Möglichkeit, daß diese beiden Gene so in dem HCV-RNA-Konstrukt angeordnet sind, daß ihre beiden exprimierten Proteine zunächst über eine Schnittstelle für eine Protease (z.B. Ubiquitin) oder über ein selbstspaltendes Peptid (z.B. das 2A-Protein der Picornaviren) fusioniert sind und erst später proteolytisch wieder getrennt werden.

Ebensogut können diese beiden Positionen aber auch derart getrennt voneinander liegen, daß beide Genprodukte separat exprimiert werden. (z.B. in der Reihenfolge: Marker- bzw. Resistenzgen ― interne Ribosomenbindungsstelle ― Reportergen).
Im Fall des Reportergens hat sich eine Ausführungsvariante besonders bewährt, bei der das Reportergen in das offene Leseraster des HCV-Genoms oder -Subgenoms einkloniert ist, und zwar derart, daß es erst nach einer proteolytischen Prozessierung in eine aktive Form überführt wird.

Das erfindungsgemäße HCV-RNA-Konstrukt für sich genommen kann in allen seinen Variationen ebenfalls für vielfältige Zwecke eingesetzt werden. Dazu gehören vor allem:
- Die Konstruktion attenuierter Hepatitis C Viren bzw. HCV-ähnlicher Partikel und deren Produktion in Zellkulturen:
   Durch zufällige oder gezielt hervorgerufene Mutationen, beispielsweise Punktmutationen, Deletionen oder Insertionen, können attenuierte HCV- oder HCV-ähnliche Partikel erzeugt werden, d.h. Viren bzw. virusähnliche Partikel mit voller Replikationskompetenz aber verringerter bzw. fehlender Pathogenität. Solche attenuierte HCV- oder HCV-ähnliche Partikel sind insbesondere als Impfstoff einsetzbar.
- Die Konstruktion von HCV-RNA-Konstrukten mit integrierten Fremdgenen, beispielsweise zur Verwendung als leberzellspezifische Genfähren in der Gentherapie. Auf Grund des ausgeprägten Leberzelltropismus des HCV und der Möglichkeit, Teile des Genoms durch heterologe Sequenzen zu ersetzen, lassen sich HCV-RNA-Konstrukte herstellen, bei denen beispielsweise die Strukturproteine durch ein therapeutisch wirksames Gen ersetzt werden. Das so erhaltene HCV-RNA-Konstrukt wird in Zellen eingeschleust, vorzugsweise mittels Transfektion, die die fehlenden HCV-Funktionen, beispielsweise die Struturproteine, konstitutiv oder induzierbar exprimieren. Durch diese dem Fachmann unter dem Begriff der 'Transkomplementation' bekannte Technik lassen sich Viruspartikel erzeugen, in die das HCV-RNA-Konstrukt eingebaut wird. Die so erhaltenen Partikel können für die Infektion vorzugsweise von Leberzellen verwendet werden. In diesen wird das therapeutisch wirksame Fremdgen zur Expression gebracht und entfaltet damit seine therapeutische Wirkung.
- Das Auffinden permissiver Zellen, d.h. Zellen, in denen eine produktive Virusvermehrung erfolgt. Zu diesem Zweck wird entweder eines der vorgenannten HCV-RNA-Genomkonstrukte verwendet, das zur Bildung kompletter infektiöser Viren befähigt ist, oder es wird eines der vorgenannten HCV-Subgenom-Konstrukte eingesetzt, das allerdings zunächst gemäß vorgenanntem Beispiel in eine Zellinie transfiziert wird, die die fehlenden Funktionen konstitutiv oder induzierbar exprimiert. In all diesen Fällen entstehen Viruspartikel, die zusätzlich zur HCV-Sequenz ein Resistenz- und/oder Reportergen tragen. Zum Auffinden von Zellen, in denen das HCV replizieren kann, werden diese Zellen mit den so hergestellten Viren infiziert und einer Antibiotikumselektion unterzogen oder, in Abhängigkeit vom HCV-RNA-Konstrukt, mittels Nachweis der Expression des Reportergens untersucht. Da eine Antibiotikumresistenz bzw. eine Expression des Reportergens nur dann nachweisbar ist, wenn das HCV-RNA-Konstrukt repliziert, müssen die so gefundenen Zellen permissiv sein. Auf diese Weise lassen sich nahezu beliebige Zellinien oder primäre Zellkulturen hinsichtlich der Permissivität testen und auffinden.

Eine andere Variante des erfindungsgemäßen HCV-RNA-Konstrukts ist dadurch gekennzeichnet, daß das HCV-RNA-Konstrukt ein integriertes Fremdgen aufweist und dazu geeignet ist, dieses Fremdgen in eine Zielzelle einzuschleusen, die zur Expression dieses Fremdgens geeignet ist.

Eine weitere Variante des erfindungsgemäßen HCV-RNA-Konstrukts zeichnet sich dadurch aus, daß das HCV-RNA-Konstrukt Nukleotid- und/oder Aminosäure-Mutationen aufweist, zellkultur-adaptiert ist, mit hoher Effizienz repliziert und dadurch erhältlich ist, daß man ein erfindungsgemäßes Zellkultursystem gemäß Anspruch 1, bei dem das eingeschleuste HCV-spezifische Genmaterial ein erfindungsgemäßes HCV-RNA-Konstrukt mit Selektionsgen nach einem der Ansprüche 1 bis 6 ist, auf/in dem dem Selektionsgen entsprechenden Selektionsmedium kultiviert, daß man die gewachsenen Zellklone erntet, und daß man aus diesen Zellklonen das HCV-RNA-Konstrukt oder Teile davon isoliert.
Eine Weiterbildung dieser Variante besteht darin, daß man das aus den Zellklonen isolierte HCV-RNA-Konstrukt oder Teile davon wenigstens einmal erneut passagiert, nämlich in Zellen des erfindungsgemäßen Zellkultursystems nach Anspruch 11 einschleust und diese Zellen auf/in dem dem Selektionsgen entsprechenden Selektionsmedium zu kultivieren, daß man die gewachsenen Zellklone erntet, und daß man aus diesen Zellklonen das HCV-RNA-Konstrukt oder Teile davon isoliert.

Ein ganz bevorzugtes HCV-RNA-Konstrukt mit hoher und sehr hoher Replikationseffizienz und infolgedessen sehr guter Eignung für die praktische Anwendung ist dadurch gekennzeichnet, daß es einen oder mehrere oder alle der in Tabelle 3 aufgelisteten Aminosäure- bzw. Nukleotidaustausche und/oder einen oder mehrere der nachfolgend aufgelisteten Aminosäureaustausche aufweist: 1283 arg -> gly , 1383 glu -> ala , 1577 lys -> arg, 1609 lys -> glu , 1936 pro -> ser , 2163 glu -> gly , 2330 lys -> glu , 2442 ile -> val. (Die Zahlen beziehen sich auf die Aminosäurepositionen des Polyproteins des HCV-Isolats con1, siehe Tabelle 1).

Eine bevorzugte Variante des erfindungsgemäßen Zellkultursystems, die sich in der Praxis sehr gut bewährt hat, ist unter der Nummer DSM ACC2394 (Laborbezeichnung HuBl 9-13) bei der DSMZ, Deutsche Sammlung von Mikroorganismen und Zellkulturen GmbH in Braunschweig, Deutschland, hinterlegt.

Mit dem erfindungsgemäßen Zellkultursystem wird erstmals ein in-vitro-System bereit gestellt, in dem HCV-RNA intrazellulär, autonom und in ausreichend großen Mengen repliziert und exprimiert wird, so daß eine quantitative Bestimmung sowohl der HCV-RNA-Mengen als auch der HCV-spezifischen Proteine mit konventionellen und zuverlässig genauen biochemischen Meßmethoden durchgeführt werden kann. Das heißt: es steht erstmals ein annähernd authentisches zellgestütztes ("cell-based") HCV-Replikationssystem zur Verfügung, das für die Entwicklung und Erprobung von antiviralen Pharmazeutika dringend benötigt wird. Dieses Testsystem bietet nun die Möglichkeit, potentielle Angriffsziele für eine wirksame HCV-spezifische Therapie zu identifizieren und HCV-spezifische Chemotherapeutika zu entwickeln und zu evaluieren.

Die Erfindung basiert auf der überraschenden Erkenntnis, daß eine effiziente Replikation der HCV-RNA nur dann in Zellen stattfindet, wenn diese mit einem HCV-RNA-Konstrukt transfiziert wurden, das mindestens die 5' und die 3' nicht-translatierten Regionen (NTR) und die Nichtstrukturproteine (NS) 3 bis 5B umfaßt und zusätzlich ein selektierbares Markergen (Selektionsgen) aufweist. Offensichtlich sind die Strukturgene für den Ablauf der Replikation ohne wesentliche Bedeutung, während andererseits eine effiziente Replikation der HCV-RNA anscheinend nur dann stattfindet, wenn die transfizierten Zellen einem permanenten Selektionsdruck unterzogen werden, der durch das mit der HCV-RNA verbundene selektierbare Markergen (Selektionsgen) vermittelt wird. Das Markergen (Selektionsgen) scheint somit einerseits die Selektion derjenigen Zellen zu provozieren, in denen die HCV-RNA produktiv repliziert, und andererseits scheint es die Effizienz der RNA-Replikation wesentlich zu steigern.

Das erfindungsgemäße Zellkultursystem in allen seinen Variationen kann für vielfältige Zwecke eingesetzt werden. Diese umfassen:
- Das Auffinden antiviral wirksamer Substanzen. Dies können beispielsweise sein: organische Verbindungen, die unmittelbar oder mittelbar in die Virusvermehrung eingreifen (z.B. Inhibitoren der viralen Proteasen, der NS3-Helikase, der NS5B RNA-abhängigen RNA Polymerase), antisense Oligonukleotide, die an eine beliebige Zielsequenz innerhalb des HCV-RNA-Konstrukts (z.B die 5' NTR) hybridisieren und unmittelbar oder mittelbar zu einer Beeinflussung der Virusvermehrung führen z.B. auf Grund einer Reduktion der Translation des HCV-Polyproteins oder Ribozyme, die eine beliebige HCV-RNA-Sequenz spalten und damit die Virusreplikation beeinträchtigen.
- Die Evaluierung jeglicher Art antiviral wirksamer Substanzen in Zellkultur. Solche Substanzen können beispielsweise mittels 'rational drug design' oder 'highthroughput screening' am isolierten gereinigten Enzym gefunden werden. Unter Evaluierung sind vor allem die Bestimmung der inhibitorischen Eigenschaften der entsprechenden Substanz sowie deren Wirkungsmechanismus zu verstehen.
- Die Identifikation neuer Angriffsziele, viralen oder zellulären Ursprungs, für eine HCV-spezifische antivirale Therapie. Ist beispielsweise ein zelluläres Protein essentiell für die Virusreplikation, kann mittels Hemmung dieses zellulären Proteins die Virusreplikation ebenfalls beeinflußt werden. Das Auffinden solcher auxiliären Faktoren ist mit dem erfindungsgemäßen System ebenfalls möglich.
- Der Einsatz für die Resistenzbestimmung. Es ist anzunehmen, daß auf Grund der hohen Mutationsrate des HCV-Genoms Therapieresistenzen auftreten. Solche Resistenzen, die gerade bei der klinischen Zulassung einer Substanz von großer Bedeutung sind, lassen sich mit dem erfindungsgemäßen Zellkultursystem ermitteln. Zellinien in denen sich das HCV-RNA-Konstrukt bzw. das HCV-Genom oder - Subgenom repliziert, werden mit steigenden Konzentrationen der entsprechenden Substanz inkubiert und die Replikation der viralen RNA wird entweder anhand eines eingebrachten Reporters oder durch qualitative oder quantitative Bestimmung der viralen Nukleinsäuren oder Proteine bestimmt. Resistenz ist dann gegeben, wenn bei normaler Wirkstoffkonzentration keine Hemmung der Replikation zu beobachten ist. Durch Reklonierung der HCV-RNA (z.B. mittels RT-PCR) und Sequenzanalyse können die für Therapieresistenz verantwortlichen Nukleotid- bzw. Aminosäureaustausche ermittelt werden. Durch Einklonieren der/des entsprechenden Austausche/s in das Ursprungskonstrukt kann deren Kausalität für die Therapieresistenz bewiesen werden.
- Die Produktion von authentischen Virusproteinen (Antigene) für die Entwicklung und/oder Evaluierung von Diagnostika. Das erfindungsgemäße Zellkultursystem erlaubt auch die Expression von HCV-Antigenen in Zellkulturen. Diese Antigene können prinzipiell auch für den Aufbau diagnostischer Nachweisverfahren eingesetzt werden.
- Die Produktion von HCV Viren und virus-ähnlichen Partikeln insbesondere zur Entwicklung oder Herstellung von Therapeutika und Impfstoffen sowie für diagnostische Zwecke. Insbesondere zellkultur-adaptierte vollständige HCV-Genome, die mit dem erfindungsgemäßen Zellkultursystem hergestellt werden können, sind in der Lage, mit hoher Effizienz in Zellkulturen zu replizieren. Diese Genome besitzen alle Funktionen des HCV und sind deshalb in der Lage infektiöse Viren zu produzieren.

Die Erfindung betrifft deshalb auch die Verwendung eines erfindungsgemäßen Zellkultursystems und/oder eines erfindungsgemäßen HCV-RNA-Konstrukts zur Herstellung und/oder Evaluierung und/oder Testung von Therapeutika und/oder Diagnostika zur Behandlung von insbesondere HCV-Infektionen sowie zur Herstellung eines Impfstoffes gegen HCV-Infektionen.

Die Erfindung betrifft ferner die Verwendung eines erfindungsgemäßen HCV-RNA-Konstrukts zur Herstellung einer leberzellspezifischen Genfähre für die Gentherapie.

Das erfindungsgemäße Zellkultursystem erlaubt auch das gezielte Auffinden von HCV-RNA-Konstrukten, bei denen es auf Grund von Mutationen, die sich entweder zufällig im Rahmen der HCV-RNA-Replikation ereignen oder die gezielt in das Konstrukt eingeführt werden, zu einer Steigerung der Replikationseffizienz kommt. Solche Mutationen, die zu einer Veränderung der Replikation des HCV-RNA-Konstrukts führen, sind dem Fachmann als adaptive Mutationen bekannt.
Die Erfindung umfaßt deshalb auch die Verwendung eines erfindungsgemäßen HCV-RNA-Konstrukts nach einem der Ansprüche 1 bis 6 zur Gewinnung von zellkultur-adaptierten Mutanten eines erfindungsgemäßen HCV-RNA-Konstrukts gemäß vorstehender Beschreibung, wobei die Mutanten gegenüber dem originären HCV-RNA-Konstrukt eine erhöhte Replikationseffizienz aufweisen. Diese Verwendung ist dadurch gekennzeichnet, daß man ein Zellkultursystem gemäß Anspruch 11 oder 12, das HCV-RNA-Konstrukte gemäß einem der Ansprüche 1 bis 6 enthält, auf/in dem dem Selektionsgen entsprechenden Selektionsmedium kultiviert, daß man die gewachsenen Zellklone erntet, und daß man aus diesen Zellklonen die HCV-RNA-Konstrukte oder Teile davon isoliert. Eine vorteilhafte Variante dieser Verwendung besteht darin, daß man die isolierten HCV-RNA-Konstrukte wenigstens einmal erneut passagiert, nämlich in Zellen eines erfindungsgemäßen Zellkultursystems einschleust und diese auf/in dem dem Selektionsgen entsprechenden Selektionsmedium kultiviert, die gewachsenen Zellklone erntet und aus diesen Zellklonen die HCV-RNA-Konstrukte oder Teile davon isoliert. Mit dieser Verfahrensvariante kann der Grad der adaptiven Mutationen und damit der Grad der Replikationseffizienz in den betreffenden HCV-RNA-Konstrukten noch gesteigert werden.
Die erfindungsgemäß hergestellten zellkultur-adaptierten HCV-RNA-Konstrukte mit hoher Replikationseffizienz sind dadurch gekennzeichnet, daß sie durch Nukleotidund/oder Aminosäureaustausche von einem HCV-RNA-Konstrukt nach einem der Ansprüche 1 bis 6 ableitbar sind und daß sie mit einem der beiden vorstehend genannten Herstellungsverfahren erhältlich sind.

Die Erfindung umfaßt desweiteren die Verwendung eines HCV-RNA-Konstrukts nach einem der Ansprüche 1 bis 6 zur Herstellung von Mutanten eines HCV-RNA-Vollängengenoms oder eines HCV-RNA-Teilgenoms oder eines beliebigen HCV-RNA-Konstrukts mit im Vergleich zu dem ursprünglichen HCV-RNA-Vollängengenom oder - Teilgenom oder HCV-RNA-Konstrukt erhöhter Replikationseffizienz. Diese Verwendung ist dadurch gekennzeichnet, daß man gemäß Anspruch 16 oder Anspruch 17 eine zellkultur-adaptierte Mutante des HCV-RNA-Konstrukts herstellt, daß man die Nukleotid- und Aminosäuresequenz dieser Mutante bestimmt und durch Vergleich mit der Nukleotid- und Aminosäuresequenz des ursprünglichen HCV-RNA-Konstrukts die Art, Anzahl und Positionen der Nukleotid- und Aminosäuremutationen bestimmt, und daß man diese Mutationen entweder durch gezielte Mutagenese oder durch Austausch von Sequenzabschnitten, die die betreffenden Mutationen enthalten, in ein (isoliertes) HCV-Vollängengenom oder ein HCV-Teilgenom oder ein beliebiges HCV-RNA-Konstrukt einführt.

Zum Nachweis bzw. zur Verifizierung derjenigen Mutationen, die tatsächlich eine Veränderung der Replikation und insbesondere eine Replikationssteigerung bewirken, kann ein Test durchgeführt werden, bei dem die bestimmten Nukleotid- und/oder Aminosäureaustausche in das ursprüngliche HCV-RNA-Konstrukt eingeführt und dieses wiederum in Zellkultur eingeschleust wird. Wenn die eingeführte Mutation tatsächlich zu einer Steigerung der Replikation führt, sollte im Fall eines HCV-RNA-Konstrukts mit selektierbarem Markergen die Zahl der resistenten Zellklone bei dem künstlich mutierten Konstrukt deutlich höher sein als bei dem unbehandelten Konstrukt. Im Fall eines Konstrukts mit einem Reportergen sollte die Aktivität bzw. Menge des Reporters bei dem künstlich mutierten Konstrukt deutlich höher sein als bei dem unbehandelten.

Diese zellkultur-adaptierten HCV-RNA-Konstrukte können dazu verwendet werden, beliebige HCV-RNA-Konstrukte oder HCV-Vollängen- oder Teilgenome mit erhöhter Replikationseffizienz herzustellen. Dabei können sowohl Konstrukte mit einem selektierbaren Resistenzgen als auch Konstrukte ohne ein solches bzw. mit einem nichtselektierbaren Reportergen (z.B. Luziferase) hergestellt werden, denn aufgrund der sehr hohen Replikationseffizienz des zellkultur-adaptierten HCV-RNA-Konstrukts kann dessen Replikation auch in nicht-selektionierten Zellen nachgewiesen werden.
Die erfindungsgemäßen zellkultur-adaptierten Mutanten eines HCV-RNA-Konstrukts oder eines HCV-Vollängengenoms oder eines HCV-Teilgenoms mit im Vergleich zu dem ursprünglichen HCV-RNA-Konstrukt oder dem ursprünglichen HCV-Vollängengenom erhöhter Replikationseffizienz , sind dadurch charakterisiert, daß sie mit einem Verfahren erhältlich sind, bei dem man in einem zellkultur-adaptierten HCV-RNA-Konstrukt durch Sequenzanalyse und Sequenzvergleich die Art und Anzahl der Mutationen bestimmt und diese Mutationen in ein HCV-RNA-Konstrukt, insbesondere in ein HCV-RNA-Konstrukt gemäß einem der Ansprüche 4 bis 19, oder in ein (isoliertes) HCV-RNA-Vollängengenom einführt, entweder durch gezielte Mutagenese oder durch Austausch von Sequenzabschnitten, die die betreffenden Mutationen enthalten.

Nicht zuletzt betrifft die Erfindung auch die Verwendung eines HCV-RNA-Konstrukts nach einem der Ansprüche 1 bis 10 zur Erzeugung von Hepatitis C Viruspartikeln oder virus-ähnlichen Partikeln.

### Besondere Eigenschaften der in den Sequenzprotokollen angegebenen Sequenzen:

**SEQ ID-NO: 1**
   Name: I389/Core-3'/wt
   Aufbau (Nukleotidpositionen):
   1. 1-341: HCV 5' nicht-translatierte Region
   2. 342-1193: HCV Core Protein-Neomycin Phosphotransferase Fusionsprotein; selektionierbarer Marker
   3. 1202-1812: Interne Ribosomenbindungsstelle des Encephalomyokarditis Virus; erlaubt die Translation des dahinterliegenden HCV offenen Leserasters
   4. 1813-10842: HCV Polyprotein von Core bis Nichtstrukturprotein 5B
   5. 1813-2385: HCV Core Protein; Strukturprotein
   6. 2386-2961: Hüllprotein 1 (envelope protein 1); Strukturprotein
   7. 2962-4050: Hüllprotein 2 (envelope protein 2); Strukturprotein
   8. 4051-4239: Protein p7
   9. 4240-4890: Nichtstrukturprotein 2 (NS2); HCV NS2-3 Protease
   10. 4891-6783: Nichtstrukturprotein 3 (NS3); HCV NS3 Protease/Helikase
   11. 6784-6945: Nichtstrukturprotein 4A (NS4A); NS3 Protease Kofaktor
   12. 6946-7728: Nichtstrukturprotein 4B (NS4B)
   13. 7729-9069: Nichtstrukturprotein 5A (NS5A)
   14. 9070-10842: Nichtstrukturprotein 5B (NS5B); RNA-abhängige RNA-Polymerase
   15. 10846-11076: HCV 3' nicht-translatierte Region
**SEQ ID-NO: 2**
   Name: I337/NS2-3'/wt
   Aufbau (Nukleotidpositionen):
   1. 1-341: HCV 5' nicht-translatierte Region
   2. 342-1181: HCV Core Protein-Neomycin Phosphotransferase Fusionsprotein; selektionierbarer Marker
   3. 1190-1800: Interne Ribosomenbindungsstelle des Encephalomyokarditis Virus; erlaubt die Translation des dahinterliegenden HCV offenen Leserasters
   4. 1801-8403: HCV Polyprotein von Nichtstrukturprotein 2 bis Nichtstrukturprotein 5B
      5. 1801-2451: Nichtstrukturprotein 2 (NS2); HCV NS2-3 Protease
      6. 2452-4344: Nichtstrukturprotein 3 (NS3); HCV NS3 Protease/Helikase
      7. 4345-4506: Nichtstrukturprotein 4A (NS4A); NS3 Protease Kofektor
      8. 4507-5289: Nichtstrukturprotein 4B (NS4B)
      9. 5290-6630: Nichtstrukturprotein 5A (NS5A)
      10. 6631-8403: Nichtstrukturprotein 5B (NS5B); RNA-abhängige RNA-Polymerase
      11. 8407-8637: HCV 3' nicht-translatierte Region
**SEQ ID-NO: 3**
   Name: I389/NS3-3'/wt
   Aufbau (Nukleotidpositionen):
   1. 1-341: HCV 5' nicht-translatierte Region
   2. 342-1193: HCV Core Protein-Neomycin Phosphotransferase Fusionsprotein; selektionierbarer Marker
   3. 1202-1812: Interne Ribosomenbindungsstelle des Encephalomyokarditis Virus; erlaubt die Translation des dahinterliegenden HCV offenen Leserasters
   4. 1813-7767: HCV Polyprotein von Nichtstrukturprotein 3 bis Nichtstrukturprotein 5B
      5. 1813-3708: Nichtstrukturprotein 3 (NS3); HCV NS3 Protease/Helikase
      6. 3709-3870: Nichtstrukturprotein 4A (NS4A); NS3 Protease Kofaktor
      7. 3871-4653: Nichtstrukturprotein 4B (NS4B)
      8. 4654-5994: Nichtstrukturprotein 5A (NS5A)
      9. 5995-7767: Nichtstrukturprotein 5B (NS5B); RNA-abhängige RNA-Polymerase
      10. 7771-8001: HCV 3' nicht-translatierte Region
**SEQ ID-NO: 4**
   Name: I337/NS3-3'/wt
   Aufbau (Nukleotidpositionen):
   1. 1-341: HCV 5' nicht-translatierte Region
   2. 342-1181: HCV Core Protein-Neomycin Phosphotransferase Fusionsprotein; selektionierbarer Marker
   3. 1190-1800: Interne Ribosomenbindungsstelle des Encephalomyokarditis Virus; erlaubt die Translation des dahinterliegenden HCV offenen Leserasters
   4. 1801-7758: HCV Polyprotein von Nichtstrukturprotein 3 bis Nichtstrukturprotein 5B
      5. 1801-3696: Nichtstrukturprotein 3 (NS3); HCV NS3 Protease/Helikase
      6. 3697-3858: Nichtstrukturprotein 4A (NS4A); NS3 Protease Kofaktor
      7. 3859-4641: Nichtstrukturprotein 4B (NS4B)
      8. 4642-5982: Nichtstrukturprotein 5A (NS5A)
      9. 5983-7755: Nichtstrukturprotein 5B (NS5B); RNA-abhängige RNA-Polymerase
      10. 7759-7989: HCV 3' nicht-translatierte Region
**SEQ ID-NO: 5**
   Name: I389/NS2-3'/wt
   Aufbau (Nukleotidpositionen):
   1. 1-341: HCV 5' nicht-translatierte Region
   2. 342-1193: HCV Core Protein-Neomycin Phosphotransferase Fusionsprotein; selektionierbarer Marker
   3. 1202-1812: Interne Ribosomenbindungsstelle des Encephalomyokarditis Virus; erlaubt die Translation des dahinterliegenden HCV offenen Leserasters
   4. 1813-8418: HCV Polyprotein von Nichtstrukturprotein 2 bis Nichtstrukturprotein 5B
      5. 1813-2463: Nichtstrukturprotein 2 (NS2); HCV NS2-3 Protease
      6. 2464-4356: Nichtstrukturprotein 3 (NS3); HCV NS3 Protease/Helikase
      7. 4357-4518: Nichtstrukturprotein 4A (NS4A); NS3 Protease Kofaktor
      8. 4519-5301: Nichtstrukturprotein 4B (NS4B)
      9. 5302-6642: Nichtstrukturprotein 5A (NS5A)
      10. 6643-8415: Nichtstrukturprotein 5B (NS5B); RNA-abhängige RNA-Polymerase
      11. 8419-8649: HCV 3' nicht-translatierte Region
**SEQ ID-NO: 6**
   Name: I389/NS3-3'/9-13F
   Aufbau (Nukleotidpositionen):
   1. 1-341: HCV 5' nicht-translatierte Region
   2. 342-1193: HCV Core Protein-Neomycin Phosphotransferase Fusionsprotein; selektionierbarer Marker
   3. 1202-1812: Interne Ribosomenbindungsstelle des Encephalomyokarditis Virus; erlaubt die Translation des dahinterliegenden HCV offenen Leserasters
   4. 1813-7767: HCV Polyprotein von Nichtstrukturprotein 3 bis Nichtstrukturprotein 5B der zellkultur-adaptierten Mutante 9-13F
      5. 1813-3708: Nichtstrukturprotein 3 (NS3); HCVNS3 Protease/Helikase
      6. 3709-3870: Nichtstrukturprotein 4A (NS4A); NS3 Protease Kofaktor
      7. 3871-4653: Nichtstrukturprotein 4B (NS4B)
      8. 4654-5994: Nichtstrukturprotein 5A (NS5A)
      9. 5995-7767: Nichtstrukturprotein 5B (NS5B); RNA-abhängige RNA-Polymerase 7771-8001: HCV 3' nicht-translatierte Region
**SEQ ID-NO: 7**
   Name: I389/Core-3'/9-13F
   Aufbau (Nukleotidpositionen):
   1. 1-341: HCV 5' nicht-translatierte Region
   2. 342-1193: HCV Core Protein-Neomycin Phosphotransferase Fusionsprotein; selektionierbarer Marker
   3. 1202-1812: Interne Ribosomenbindungsstelle des Encephalomyokarditis Virus; erlaubt die Translation des dahinterliegenden HCV offenen Leserasters
   4. 1813-10842: HCV Polyprotein von Core bis Nichtstrukturprotein 5B der zellkultur-adaptierten Mutante 9-13F
      5. 1813-2385: HCV Core Protein; Strukturprotein
      6. 2386-2961: Hüllprotein 1 (envelope protein 1); Strukturprotein
      7. 2962-4050: Hüllprotein 2 (envelope protein 2); Strukturprotein
      8. 4051-4239: Protein p7
      9. 4240-4890: Nichtstrukturprotein 2 (NS2); HCV NS2-3 Protease
      10. 4891-6783: Nichtstrukturprotein 3 (NS3); HCV NS3 Protease/Helikase
      11. 6784-6945: Nichtstrukturprotein 4A (NS4A); NS3 Protease Kofaktor
      12. 6946-7728: Nichtstrukturprotein 4B (NS4B)
      13. 7729-9069: Nichtstrukturprotein 5A (NS5A)
      14. 9070-10842: Nichtstrukturprotein 5B (NS5B); RNA-abhängige RNA-Polymerase
      15. 10846-11076: HCV 3' nicht-translatierte Region
**SEQ ID-NO: 8**
   Name: I389/NS3-3'/5.1
   Aufbau (Nukleotidpositionen):
   1. 1-341: HCV 5' nicht-translatierte Region
   2. 342-1193: HCV Core Protein-Neomycin Phosphotransferase Fusionsprotein; selektionierbarer Marker
   3. 1202-1812: Interne Ribosomenbindungsstelle des Encephalomyokarditis Virus; erlaubt die Translation des dahinterliegenden HCV offenen Leserasters
   4. 1813-7767: HCV Polyprotein von Nichtstrukturprotein 3 bis Nichtstrukturprotein 5B der zellkultur-adaptierten Mutante 5.1
      5. 1813-3708: Nichtstrukturprotein 3 (NS3); HCV NS3 Protease/Helikase
      6. 3709-3870: Nichtstrukturprotein 4A (NS4A); NS3 Protease Kofaktor
      7. 3871-4653: Nichtstrukturprotein 4B (NS4B)
      8. 4654-5994: Nichtstrukturprotein 5A (NS5A)
      9. 5995-7767: Nichtstrukturprotein 5B (NS5B); RNA-abhängige RNA-Polymerase 7771-8001: HCV 3' nicht-translatierte Region
**SEQ ID-NO: 9**
   Name: I389/Core-3'/5.1
   Aufbau (Nukleotidpositionen):
   1. 1-341: HCV 5' nicht-translatierte Region
   2. 342-1193: HCV Core Protein-Neomycin Phosphotransferase Fusionsprotein; selektionierbarer Marker
   3. 1202-1812: Interne Ribosomenbindungsstelle des Encephalomyokarditis Virus; erlaubt die Translation des dahinterliegenden HCV offenen Leserasters
   4. 1813-10842: HCV Polyprotein von Core bis Nichtstrukturprotein 5B der zellkultur-adaptierten Mutante 5.1
      5. 1813-2385: HCV Core Protein; Strukturprotein
      6. 2386-2961: Hüllprotein 1 (envelope protein 1); Strukturprotein
      7. 2962-4050: Hüllprotein 2 (envelope protein 2); Strukturprotein
      8. 4051-4239: Protein p7
      9. 4240-4890: Nichtstrukturprotein 2 (NS2); HCV NS2-3 Protease
      10. 4891-6783: Nichtstrukturprotein 3 (NS3); HCV NS3 Protease/Helikase
      11. 6784-6945: Nichtstrukturprotein 4A (NS4A); NS3 Protease Kofaktor
      12. 6946-7728: Nichtstrukturprotein 4B (NS4B)
      13. 7729-9069: Nichtstrukturprotein 5A (NS5A)
      14. 9070-10842: Nichtstrukturprotein 5B (NS5B); RNA-abhängige RNA-Polymerase
      15. 10846-11076: HCV 3' nicht-translatierte Region
**SEQ ID-NO: 10**
   Name: I389/NS3-3'/19
   Aufbau (Nukleotidpositionen):
   1. 1-341: HCV 5' nicht-translatierte Region
   2. 342-1193: HCV Core Protein-Neomycin Phosphotransferase Fusionsprotein; selektionierbarer Marker
   3. 1202-1812: Interne Ribosomenbindungsstelle des Encephalomyokarditis Virus; erlaubt die Translation des dahinterliegenden HCV offenen Leserasters
   4. 1813-7767: HCV Polyprotein von Nichtstrukturprotein 3 bis Nichtstrukturprotein 5B der zellkultur-adaptierten Mutante 19
      5. 1813-3708: Nichtstrukturprotein 3 (NS3); HCV NS3 Protease/Helikase
      6. 3709-3870: Nichtstrukturprotein 4A (NS4A); NS3 Protease Kofaktor
      7. 3871-4653: Nichtstrukturprotein 4B (NS4B)
      8. 4654-5994: Nichtstrukturprotein 5A (NS5A)
      9. 5995-7767: Nichtstrukturprotein 5B (NS5B); RNA-abhängige RNA-Polymerase 7771-8001: HCV 3' nicht-translatierte Region
**SEQ ID-NO: 11**
   Name: I389/Core-3'/19
   Aufbau (Nukleotidpositionen):
   1. 1-341: HCV 5' nicht-translatierte Region
   2. 342-1193: HCV Core Protein-Neomycin Phosphotransferase Fusionsprotein; selektionierbarer Marker
   3. 1202-1812: Interne Ribosomenbindungsstelle des Encephalomyokarditis Virus; erlaubt die Translation des dahinterliegenden HCV offenen Leserasters
   4. 1813-10842: HCV Polyprotein von Core bis Nichtstrukturprotein 5B der zellkultur-adaptierten Mutante 19
      5. 1813-2385: HCV Core Protein; Strukturprotein
      6. 2386-2961: Hüllprotein 1 (envelope protein 1); Strukturprotein
      7. 2962-4050: Hüllprotein 2 (envelope protein 2); Strukturprotein
      8. 4051-4239: Protein p7
      9. 4240-4890: Nichtstrukturprotein 2 (NS2); HCV NS2-3 Protease
      10. 4891-6783: Nichtstrukturprotein 3 (NS3); HCV NS3 Protease/Helikase
      11. 6784-6945: Nichtstrukturprotein 4A (NS4A); NS3 Protease Kofaktor
      12. 6946-7728: Nichtstrukturprotein 4B (NS4B)
      13. 7729-9069: Nichtstrukturprotein 5A (NS5A)
      14. 9070-10842: Nichtstrukturprotein 5B (NS5B); RNA-abhängige RNA-Polymerase
      15. 10846-11076: HCV 3' nicht-translatierte Region

Die Erfindung wird im folgenden anhand von Ausführungsbeispielen und dazugehörigen Tabellen und Figuren näher erläutert. Die erwähnten Figuren zeigen
- **Fig. 1 A:**: Die Struktur eines erfindungsgemäßen HCV-RNA-Konstrukts
Ganz oben ist eine schematische Darstellung der Struktur des kompletten parentalen HCV-Genoms gegeben mit den Positionen der Gene für die Spaltungsprodukte core, E1, E2, p7, NS2, NS3, NS4A, NS4B, NS5A und NS5B innerhalb des Polyproteins, und den 5' und 3' nichttranslatierten Regionen (5' NTR und 3' NTR) ― als Horizontalbalken dargestellt ―, und mit den beiden für die Erzeugung der Subgenom-Konstrukte ausgewählten Positionen, nämlich der Position der 'GDD-katalytischen Domäne' der NS5B RNA Polymerase (GDD) und der Position der 3' Grenze der HCV-IRES (Nukleotidpositionen 1 bis 377 bzw. 1 bis 389) ― oberhalb des Genomschemas eingezeichnet ―. Die Zahlen unterhalb des Genomschemas bezeichnen die entsprechenden Nukleotidpositionen.
Darunter sind schematische Darstellungen der Strukturen zweier erfindungsgemäßer, modifizierter HCV-RNA-Konstrukte (Subgenom) gezeigt, bestehend aus der 5' HCV-IRES, dem Neomycinphosphotransferasegen (Neo^{R}), der EMCV-IRES (E-I) und den HCV Sequenzen von NS2 bzw. NS3 bis zum authentischen 3' Ende. Die Position der das NS5B Polymerase GDD-Motiv umfassenden 10 Aminosäuren-Deletion ist jeweils mit einem Dreieck (Δ) markiert.
- **Fig. 1 B:**: Das Ergebnis einer denaturierenden Formaldehyd-Agarose-Gelelektrophorese zum Nachweis von replizierter Plusstrang-RNA in transfizierten subpassagierten Huh-7 Zellklonen. Die Positionen der HCV-spezifischen RNAs (Pfeile) und der 28S rRNA sind rechts von Spur 12 angegeben, die Größen (Anzahlen der Nukleotide) der RNA-Marker (M) sind links von Spur 1 angegeben.
- **Fig. 1 C:**: Das Ergebnis eines PCR-Tests mit nachfolgendem Southern-Blot zum Nachweis der Abwesenheit von integrierter Replikon-DNA in den meisten der selektierten Zellklone.
Spuren 1 und 2 zeigen die Positivkontrollen, Spur 13 die Negativ-Kontrolle. Die Zahlenangaben links der Spur 1 bezeichnen die Größe der Nukleotid-Marker-Moleküle.

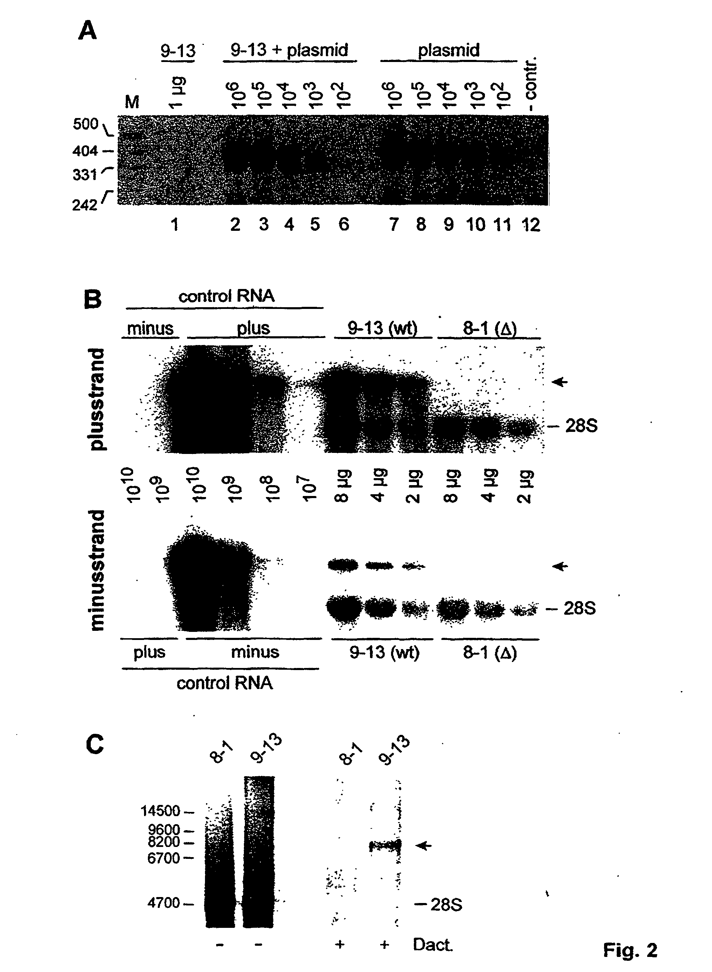

- **Fig. 2 A:**: Das Ergebnis eines PCR-Tests mit nachfolgendem Southern-Blot zum sensitiven Ausschluß integrierter Replikon-DNA (Plasmid Moleküle I₃₇₇/NS3-3'/wt) in einem HCV-RNA-Konstrukt-haltigen Zellklon (9-13). Die Spuren 7 bis 11 repräsentieren das Ergebnis einer Titration von DNA-Molekülen des Konstrukts I₃₇₇/NS3-3'/wt ohne Zugabe von Gesamt-DNA des Zellklons 9-13 und die Spuren 2 - 6 repräsentieren die gleichen Plasmidmoleküle mit Zugabe von jeweils 1 µg 9-13 DNA vor der PCR (zwecks Ausschluß eines Inhibitors der PCR in der DNA-Präparation). Spur 13 repräsentiert die Negativ-Kontrolle (PCR ohne DNA-Sonde). Spur 1 zeigt das Ergebnis, das mit einem µg Gesamt-DNA des Zellkons 9-13 erhalten wurde.
- **Fig. 2 B:**: Das Ergebnis eines Northern-Blot-Tests zur Quantifizierung von HCV Plus-und Minusstrang RNA.
Die Pfeile markieren die Positionen von Replikon-RNA. Die "plus" und "minus" -Abgaben bezeichnen die positive (plus) bzw. negative (minus) Polarität der RNA-Kontrollen, die auf das Gel aufgetragen wurden. "Minusstrand" und "Plusstrand" bezeichnen die Spezifität der radioaktiven RNA-Sonden.
- **Fig. 2 C:**: Ergebnis einer Formaldehyd-Agarose-Gelelektrophorese nach radioaktiver Markierung der intrazellulär replizierten HCV-RNA zum Nachweis der Resistenz der HCV-RNA-Replikation gegen Dactinomycin.

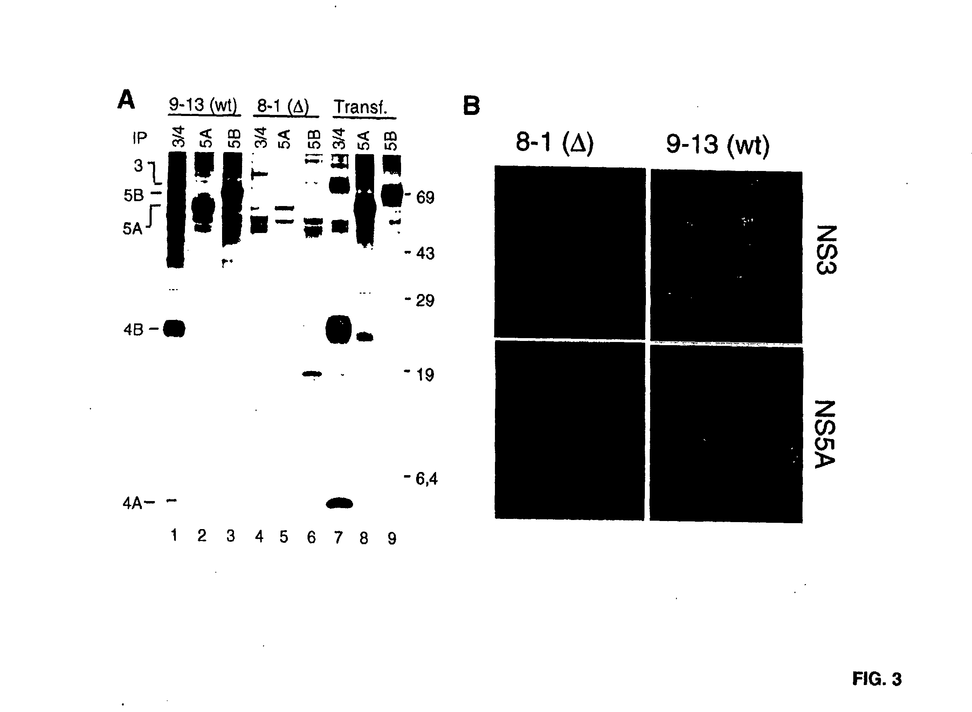

- **Fig. 3 A:**: Nachweis von HCV-spezifischen Antigenen in den selektierten Zellklonen mittels Immunopräzipitation nach metabolischer Radioaktivmarkierung. Die Spuren 7 - 9 repräsentieren authentische Größenmarker (die nach transienter Expression eines HCV-RNA-Konstrukts in Huh-7-Zellen erhalten wurden); identifizierte HCV-Proteine sind am linken Rand von Spur 1 markiert, die Molekulargewichte (in Kilodalton) sind am rechten Rand von Spur 9 angegeben.
- **Fig. 3 B:**: Ergebnisse eines Immunfluoreszenztests zum Nachweis der subzellulären Lokalisation von HCV Antigenen.

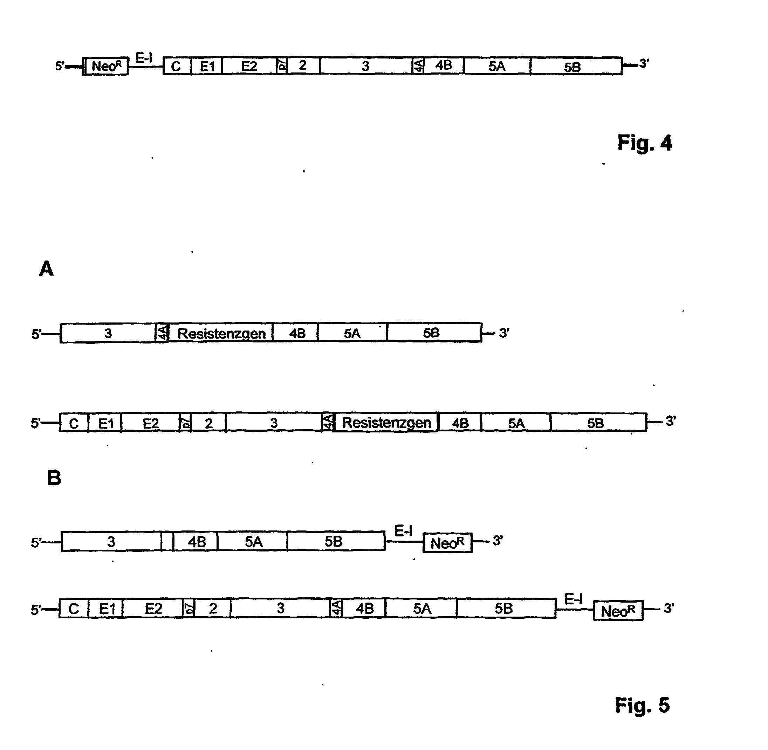

- **Fig. 4 :**: Schematische Darstellung der Struktur eines erfindungsgemäßen selektierbaren HCV-RNA-Konstrukts (komplettes Genom) bestehend aus der 5' HCV-IRES, dem Neomycinphosphotransferasegen (NeoR), einem heterologen IRES-Element, z.B. des Encephalomyocarditisvirus (E-I), dem vollständigen HCV-Leseraster und der authentischen 3' NTR.
- **Fig. 5:**: Schematische Darstellung der Struktur von HCV-RNA-Konstrukten mit insertiertem Antibiotikumresistenzgen **(A)** innerhalb der für das Polyprotein kodierenden Nukleotidsequenz (monocistronische RNA), und **(B)** innerhalb der 3' NTR (bicistronische RNA).

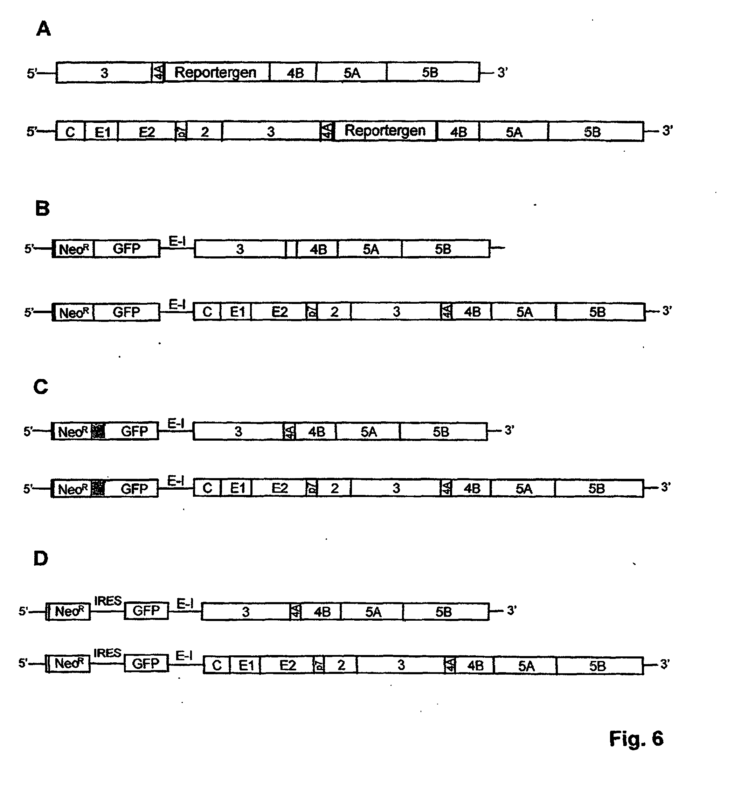

- **Fig. 6:**: Schematische Darstellung der Struktur von HCV-RNA-Konstrukten mit insertiertem Reportergen **(A)** als Teil eines HCV-Replikons von NS3 bis NS5B; ― das Reporterprotein wird letztendlich durch virale oder durch zelluläre Proteasen aus dem Polyprotein gespalten und das selektierbare Markergen (Selektionsgen) bzw. das Resistenzgen durch Kontransfektion in die Zellen einschleust , **(B)** als Teil eines Fusionsgens aus Resistenz- und Reportergen (z.B. für die Neomycinphosphotransferase und green fluorescent Protein) **(C)** als Teil eines Replikons aus Resistenz- und Reportergen (z.B. für die Neomycinphosphotransferase und das green fluorescent Protein), die über eine Nukleotidsequenz verbunden sind, welche für eine Aminosäuresequenz kodiert (schraffierter Bereich), die von einer Protease gespalten werden kann oder die über eine selbstspaltende (autokatalytische) Aktivität verfügt, **(D)** als unabhängiges Gen (hier green fluorescent protein), das von einer eigenen internen Ribosomenbindungsstelle (IRES) aus exprimiert wird; ― das Resistenzgen (hier: Neomycinphosphotransferase-Gen) wird davon unabhängig ebenfalls von einer eigenen internen Ribosomenbindungsstelle (IRES) aus exprimiert (polycistronisches Konstrukt).

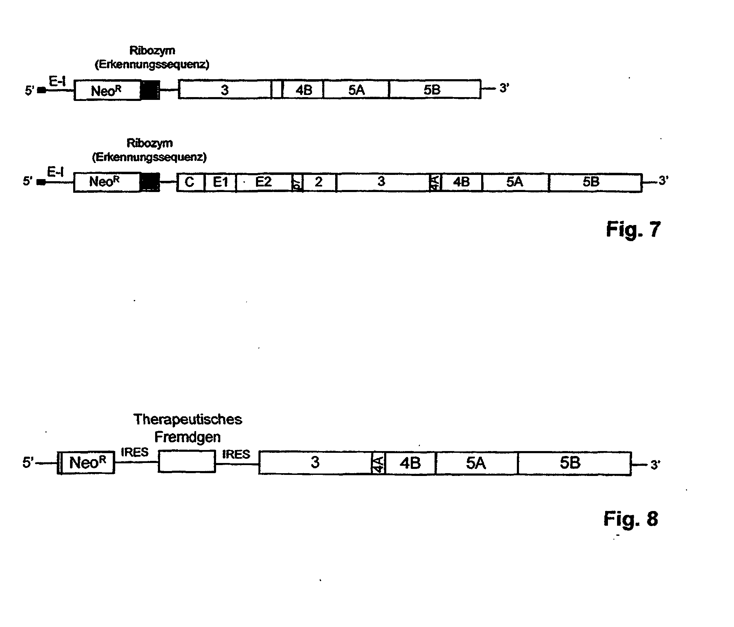

- **Fig. 7:**: Schematische Darstellung der Struktur eines HCV-RNA-Konstrukts bei dem das Resistenzgen über ein Ribozym bzw. eine Erkennungsstelle für ein Ribozym mit der HCV-RNA-Sequenz verbunden ist. Die dicken Linien stellen die HCV 5' und 3' NTRs dar, E-I ist eine heterologe interne Ribosomenbindungsstelle, die für die Expression des Resistenzgens notwendig ist, und das graue Quadrat stellt das Ribozym bzw. eine Erkennungsstelle für ein Ribozym dar.
- **Fig. 8:**: Schematische Darstellung der Struktur eines HCV-RNA-Konstrukts mit Resistenzgen und integriertem Fremdgen.

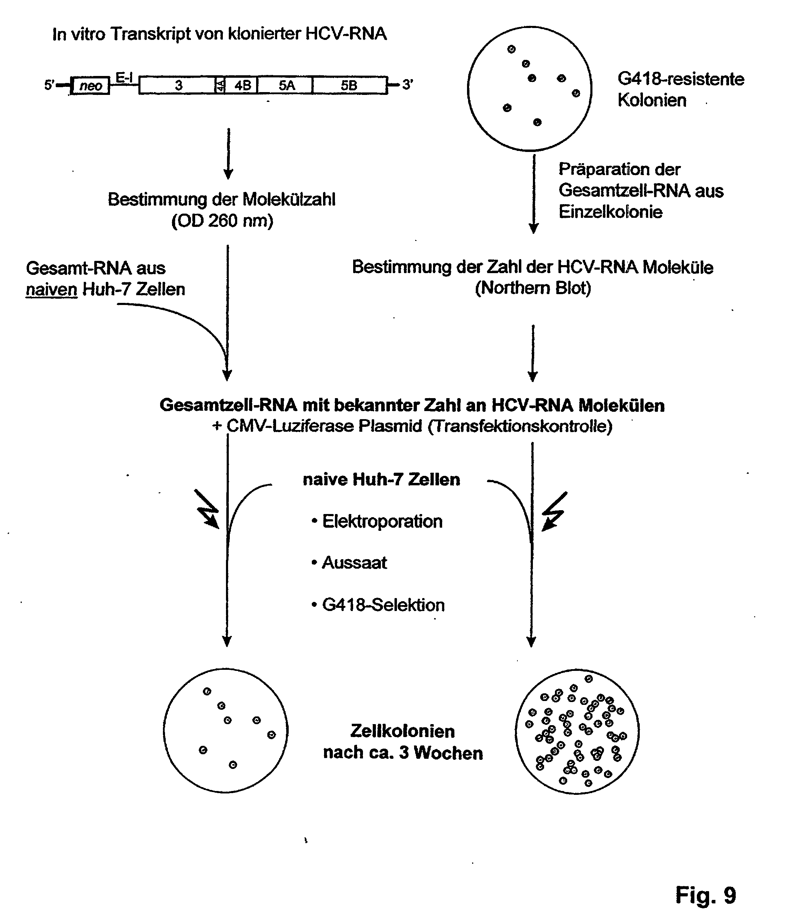

- **Fig. 9:**: Methodisches Vorgehen zum Vergleich der spezifischen Infektiosität (ausgedrückt als Anzahl gebildeter Zellkolonien) von Gesamt-RNA versus in vitro Transkripte. HCV-RNA wird mittels in vitro Transkription eines entsprechenden RNA-Konstrukts hergestellt und durch Messung der optischen Dichte bei 260 nm (OD 260 nm) quantifiziert. Eine definierte Anzahl dieser Moleküle wird mit einer bestimmten Menge Gesamt-RNA von naiven Huh-7 Zellen gemischt und diese Mischung mit Hilfe der Elektroporation in naïve Huh-7 Zellen eingeschleust. Parallel dazu wird die Gesamt-RNA eines Zellklons, der mit der in Figur 1 beschriebenen Methode hergestellt wurde, mit einem im Stand der Technik bekannten Verfahren isoliert und die Menge der darin enthaltenen HCV-RNA mittels Northernblot unter Verwendung einer HCV-spezifischen RNA-Sonde und anschließender Quantifizierung mittels Phosphoimager bestimmt. Eine definierte Menge dieser Gesamt-RNA wird analog den in vitro Transkripten in naïve Huh-7 Zellen transfiziert. Diese Zellen in beiden Ansätzen werden danach einer G418-Selektion unterzogen und die Anzahl der gebildeten Kolonien durch Auszählen nach fixieren und anfärben mit Coomassie-Brilliant-Blau bestimmt. Zur Bestimmung der Transfektionseffizienz wird jedem Transfektionsansatz 1µg eines Plasmids zugesetzt, das die Expression der Luziferase erlaubt. Ein Aliquot der transfizierten Zellen wird nach 24 Stunden geerntet und die Luziferaseaktivität im jeweiligen Zellysat bestimmt.
Die Anzahl der Kolonien wird jeweils auf die Luziferaseexpression normiert.

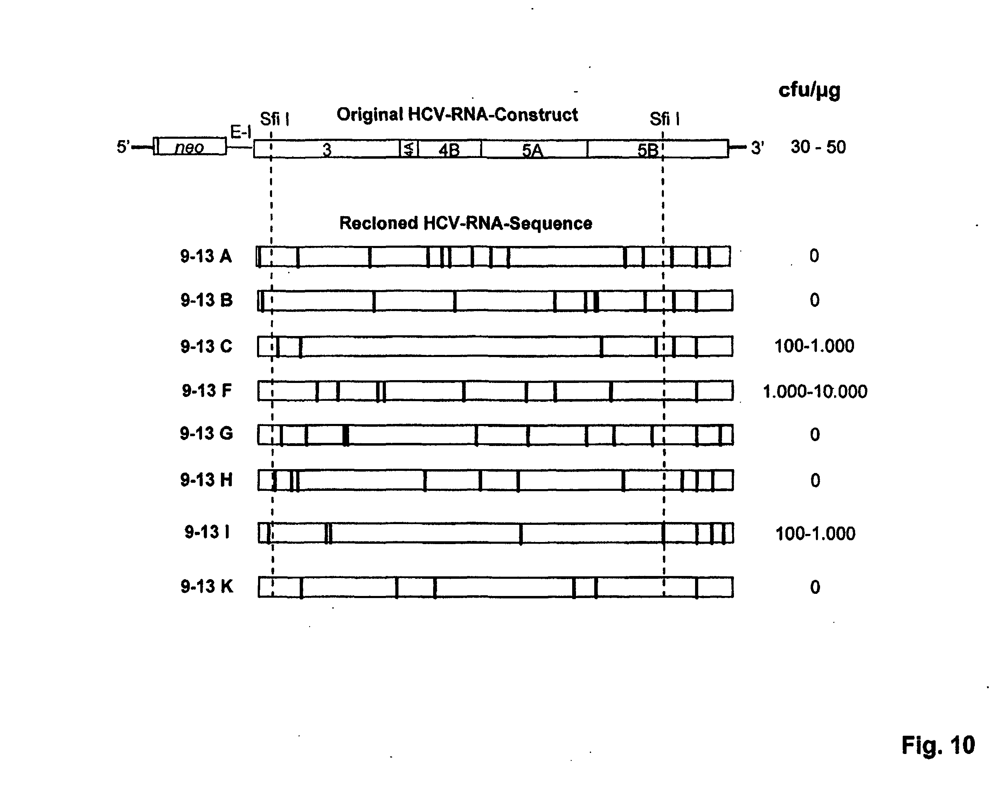

- **Fig. 10:**: Sequenzanalyse der 9-13 Klone. Gesamt-RNA des Zellklons 9-13, der durch Transfektion des HCV-RNA-Konstrukts I377/NS3-3' entstand, wurde mit einem im Stand der Technik bekannten Verfahren isoliert und das HCV-RNA-Konstrukt von Nukleotidposition 59 bis 9386 mit Hilfe der 'long-distance RT-PCR' unter Verwendung der primer S59 und A9413 amplizifiert. Die PCR-Fragmente wurden kloniert und 11 Klone (genannt 9-13 A - K) vollständig sequenziert, wobei sich die Klone D und I, E und G sowie H und J als identisch erwiesen. Die Positionen der Aminosäureunterschiede in der NS3-5B Region zwischen den reklonierten HCV-RNAs und dem parentalen Konstrukt sind mit einem dicken vertikalen Strich beim jeweiligen Klon markiert. Jeder Klon wurde mit dem Restriktionsenzym *Sfi* I verdaut und das jeweilige Fragment in das parentale Konstrukt inseriert. Diese Klone wurden jeweils in Huh-7 Zellen transfiziert und die Zellen wie in Figur 1 beschrieben einer Selektion unterzogen. Die Anzahl der mit jedem Konstrukt erhaltenen Zellklone ist rechts neben dem jeweiligen Konstrukt vermerkt.

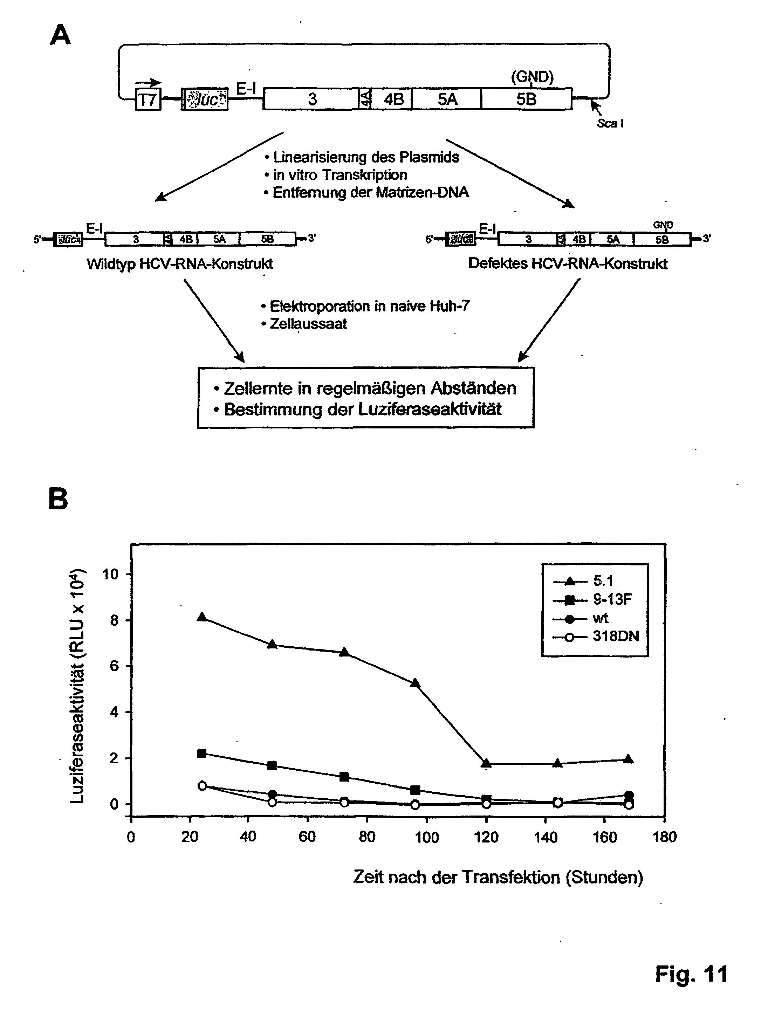

- **Fig. 11 A:**: Prinzip der Replikationsbestimmung mit Hilfe eines Reportergens. Im oberen Teil der Figur ist das HCV-DNA-Konstrukt I₃₈₉/Luc/NS3-3' dargestellt, bestehend aus der HCV 5' NTR (Nukleotidposition 1-389), dem Luziferasegen (*luc*), der IRES des Encephalomyocarditis Virus, dem HCV NS3-5B und der 3' NTR. Die Position des aktiven Zentrums der NS5B RNA-Polymerase, in das ein inaktivierender Aminosäureaustausch eingeführt wurde, ist mit 'GND' angedeutet. Die Plasmide, die fiir das replikationskompetente bzw. das defekte HCV-RNA-Konstrukt kodieren, werden mit dem Restriktionsenzym *Sca* I verdaut und in eine in vitro Transkription mit der T7 RNA-Polymerase eingesetzt. Nach Entfernung der Matrizen-DNA werden die jeweiligen HCV-RNA-Konstrukte mittels Elektroporation in naive Huh-7 Zellen eingeschleust und diese in regelmäßigen Abständen geerntet.
- **Fig. 11 B:**: Vergleich der Luziferaseaktivitäten in Zellen transfiziert mit dem parentalen HCV-RNA-Konstrukt I₃₈₉/Luc/NS3-3'/wt (wt) oder den folgenden Varianten: Der inaktiven RNA (318 DN), der Variante 9-I3F oder der Variante 5.1. Die Zellen wurden 6 (nicht gezeigt), 24, 48, 72, 96, 120, 144 und 168 Stunden nach der Transfektion geerntet und die Luziferaseaktivitäten luminometrisch bestimmt.

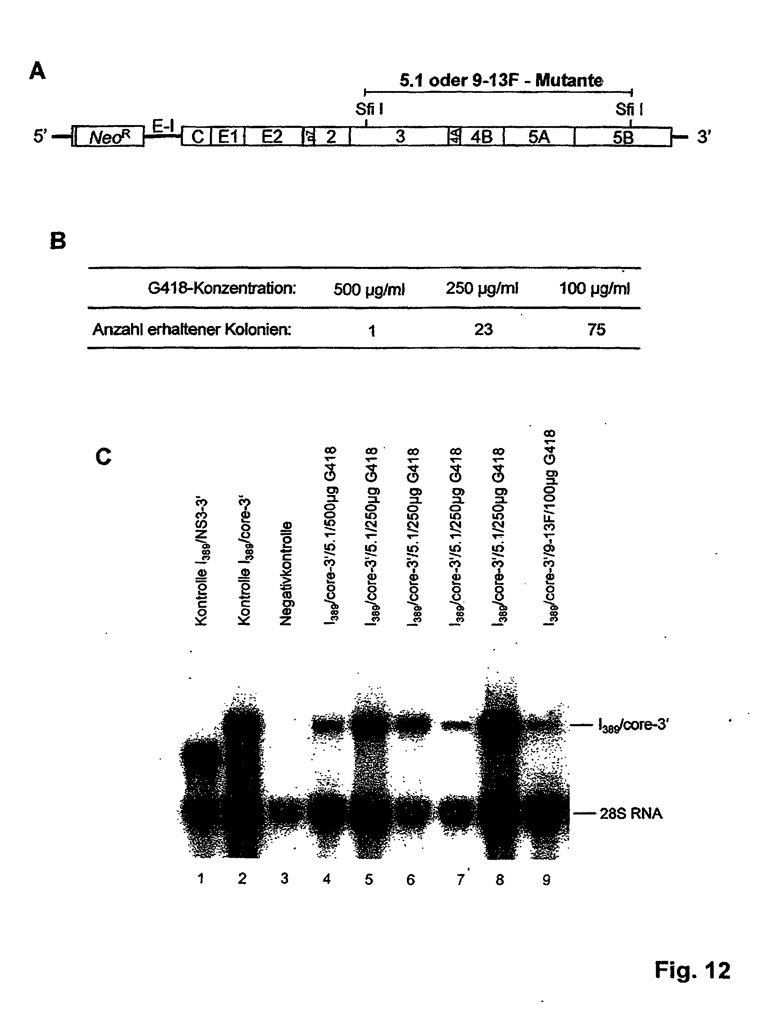

- **Fig. 12:**: Selektionierbare HCV-Vollängengenome (Konstrukte I₃₈₉/core-3'/5.1 und I₃₈₉/core-3'/9-13F).
(A) Schematische Darstellung des Vollängenkonstrukts. Der Bereich zwischen den beiden angedeuteten Erkennungsstellen für das Restriktionsenzym Sfi I entspricht den Sequenzen der hoch-adaptierten RNA-Varianten 5.1. oder 9-13F.
(B) Anzahl der Kolonien die nach Transfektion von jeweils 0,1 µg in vitro transkribierter RNA der unter A dargestellten Konstrukte I₃₈₉/core-3'/5.1 in HUH7-Zellen erhalten wurden. Angegeben ist das Ergebnis eines repräsentativen Experimentes.
(C) Nachweis autonom replizierender HCV-Vollängen-RNAs in G418-resistenten Zellklonen, die nach Transfektion des entsprechenden in vitro Transkripts erhalten wurden. Die Abbildung zeigt das Autoradiogramm eines Northern Blots, der mit einer Sonde gegen das *neo*-Resistenzgen und der HCV 5' NTR hybridisiert wurde. Die in Spur 1 und 2 dargestellten Kontrollen entsprechen jeweils 10⁸ Molekülen der angegebenen in vitro Transkripte, gemischt mit Gesamt-RNA aus naiven Huh-7 Zellen. Die Negativkontrolle enthält ausschließlich Gesamt RNA aus naiven Huh-7 Zellen (Spur 3). Die Spuren 4-9 enthalten 3-10 µg Gesamt-RNA aus G418-resistenten Zellklonen, die nach Transfektion von in vitro transkribierter I₃₈₉/core-3'/5.1-RNA bzw. I₃₈₉/core-3'/9-13F-RNA erhalten wurden. Die für die Selektion verwendete G418-Konzentration ist jeweils angegeben. Fünf der dargestellten Zellklone enthalten die hoch adaptierte RNA-Variante 5.1 (Spur 4-8), einer die adaptierte RNA-Variante 9-13F (Spur 9).

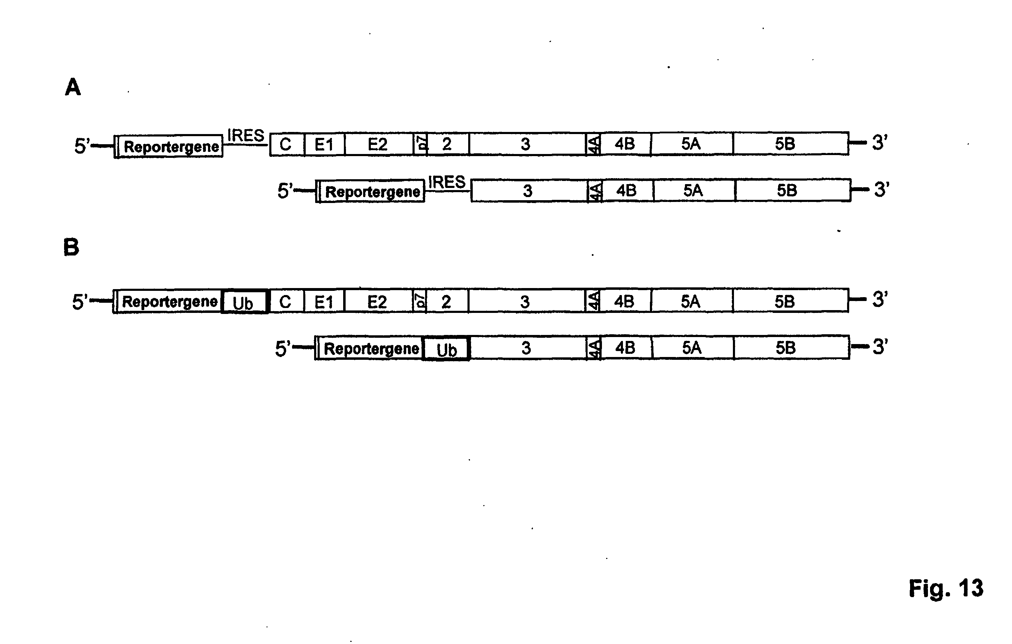

- **Fig. 13:**: HCV-RNA-Konstrukte mit einem Reportergen. (A) Bicistronische HCV-RNA-Konstrukte. Das Reportergen wird mit Hilfe einer separaten IRES translatiert. (B) Monocistronische HCV-RNA-Konstrukte. Das Reportergenprodukt wird als Fusionsprotein mit einem HCV-Protein exprimiert. Die beiden Anteile sind über eine Erkennungssequenz für eine virale oder zelluläre Protease verbunden, die eine proteolytische Trennung der beiden fusionierten Proteinanteile erlaubt. Im gezeigten Beispiel wurden das Reportergenprodukt und das jeweilige HCV-Protein über eine Erkennungssequenz für Ubiquitin (Ub) fusioniert.

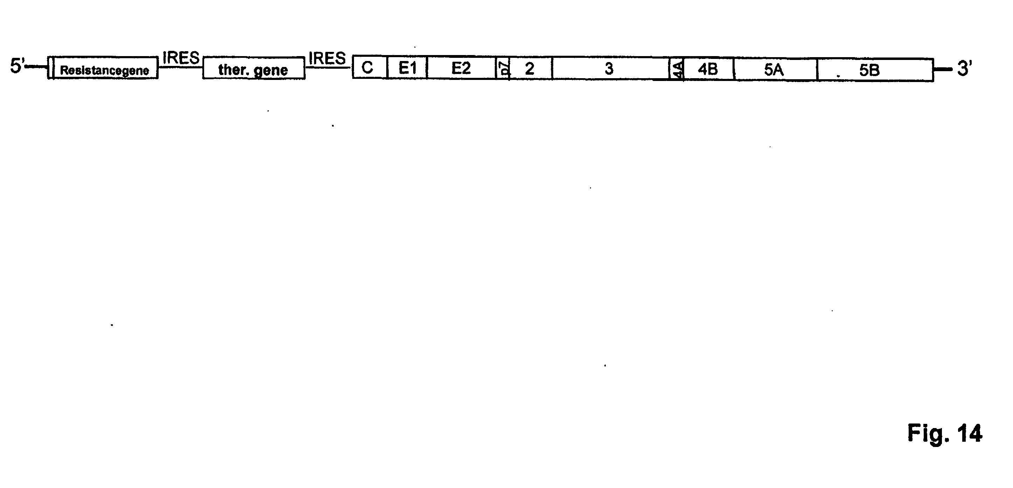

- **Fig. 14:**: Tricistronisches Vollängen HCV-RNA-Konstrukt, das zusätzlich zum Resistenzgen ein Fremdgen inseriert besitzt.

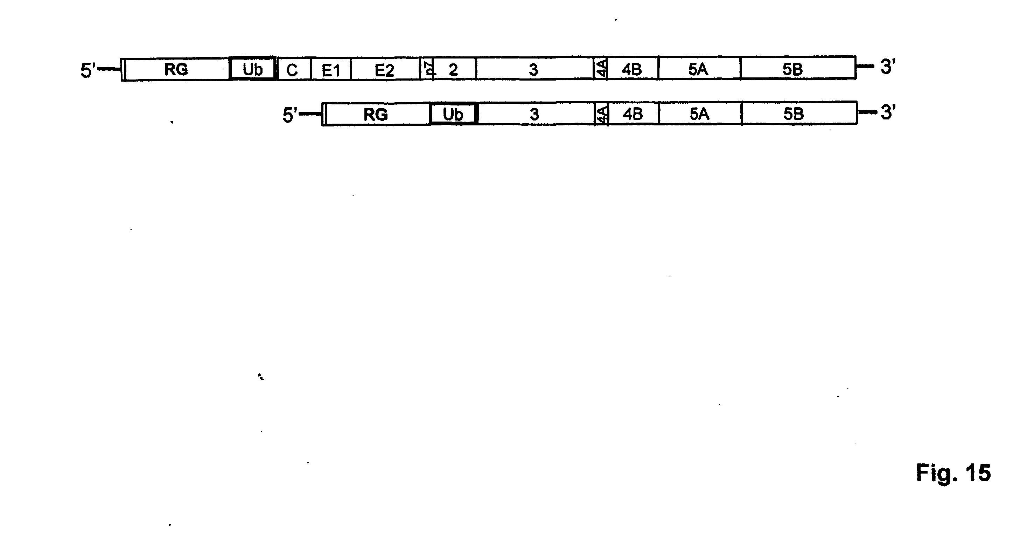

- **Fig. 15:**: Monocistronische HCV-RNA-Konstrukte, bei denen das Resistenzgenprodukt als Fusionsprotein mit dem HCV-Anteil exprimiert wird. Das Resistenzgen (RG) ist entweder als Fusionsprotein aktiv oder es wird so mit einer proteolytisch spaltbaren Sequenz mit dem HCV-Anteil fusioniert, daß das Resistenzgenprodukt durch eine zelluläre oder virale Protease vom HCV-Anteil abgespalten wird. Im gezeigten Beispiel wurde das Resistenzgen über die für Ubiquitin (Ub) kodierende Sequenz mit dem jeweiligen HCV-Anteil fusioniert.

### Beispiel 1: Herstellung von HCV-RNA-Konstrukten

### (A) Synthese und Klonierung eines vollständigen HCV-Konsensusgenoms mittels RT-PCR

Aus der Leber eines chronisch infizierten Patienten wurde das HCV-Genom, d.h die HCV-RNA wie nachfolgend beschrieben isoliert:

Aus ca. 100 mg Leber wurde die komplette RNA gemäß dem Verfahren von Chomczynski und Sacci (1987, Anal. Biochem. 162, 156) isoliert. Mit 1 µg dieser isolierten RNA wurde eine reverse Transkription mit den Primern A6103 (GCTATCAGCCGGTTCATCCACTGC) oder A9413 (CAGGATGGCCTATTGG CCTGGAG) und dem 'expand reverse transcriptase'-System (Boehringer Mannheim, Deutschland) nach den Vorschriften des Herstellers durchgeführt. Mit den Produkten dieser reversen Transkription (RT) wurde eine Polymerase-Kettenreaktion (PCR=polymerase chain reaction) durchgeführt, und zwar unter Verwendung des 'expand long template'-Systems (Boehringer Mannheim, Deutschland), wobei der Puffer mit 2% Dimethylsulfoxid-Gehalt eingesetzt wurde. Nach einer Stunde bei 42°C wurde 1/8 dieses Reaktionsansatzes in einem ersten PCR-Durchgang mit den Primern A6103 und S59 (TGTCTTCACGCAGAAAGCGTCTAG) oder A9413 und S4542 (GATGAGCT CGCCGCGAAGCTGTCC) eingesetzt. Nach 40 Zyklen wurde 1/10 dieses Reaktionsansatzes in einem zweiten PCR-Durchgang mit den Primern S59 und A4919 (AGCACAGCCCGCGTCATAGCACTCG) oder S4542 und A9386 (TTAGCTCCCCG TTCATCGGTTGG) eingesetzt. Nach 30 Zyklen wurden die PCR-Produkte mittels präparativer Agarose-Gel-Elektrophorese gereinigt und die dabei eluierten Fragmente wurden in den Vektor pCR2.1 (Invitrogen) oder pBSK II (Stratagene) ligiert. Vier Klone von jedem Fragment wurden analysiert und sequenziert, und es wurde eine Konsensus-Sequenz ermittelt. Zu diesem Zweck wurden die DNA-Sequenzen miteinander verglichen. Die Positionen, an denen sich die Sequenz eines der Fragmente von den übrigen unterschied, wurde als unerwünschte Mutation betrachtet. Im Fall von Mehrdeutigkeiten der Sequenz wurden kürzere sich überlappende PCR-Fragmente der betreffenden Region amplifiziert und mehrere Klone sequenziert. Auf diese Weise konnten zahlreiche potentielle Mutationen in jedem Fragment identifiziert und somit eine isolat-spezifische Konsensussequenz etabliert werden. Diese etablierte Konsensussequenz bzw. dieses Genom gehört zum weltweit verbreiteten Genotyp 1b. Die nicht translatierte Region am 3'-Ende (=3' NTR) wurde mittels konventioneller PCR erhalten, wobei ein Antisense-Primer eingesetzt wurde, der die letzten 24 Nukleotide des im Stand der Technik bekannten 'X-tails' (Tanaka et al., 1995, Biochem. Biophys. Res. Commun. 215, 744; und Rice, PCT/US 96/14033) abdeckt. Die authentische nicht translatierte Region am 5'-Ende (=5' NTR) strangabwärts vom T7 Promotor wurde mittels PCR erzeugt, wobei zum einen ein Oligonukleotid verwendet wurde, das einem verkürzten T7 Promotor (TAA TAC GAC TCA CTA TAG) und den ersten 88 Nukleotiden von HCV entspricht, und zum anderen eines der vorgenannten Plasmide eingesetzt wurde, das eines der 5' Fragmente des Genoms trägt. Aus den subgenomischen Fragmenten mit der geringsten Anzahl an Nicht-Konsensus-Austauschen wurde ein komplettes HCV-Konsensusgenom zusammengesetzt und in einen modifizierten pBR322-Vektor insertiert. Abweichungen von der Konsensussequenz wurden mittels ortsgerichteter Mutagenese ("site-directed mutagenesis) beseitigt. Um "run-off"-Transkripte mit einem authentischen 3' Ende herzustellen, wurde die 3'-NTR der Isolate (mit dem Ende TGT) zu AGT modifiziert (gemäß der Sequenz vom Genotyp 3 = Klon 'WS' nach Kolykhalov et al., 1996, J. Virol. 70, 3363) und außerdem wurde ein zusätzlicher Nukleotidaustausch an Position 9562 vorgenommen, um die A:T Basenpaarung in der Haarnadelstruktur am 3'Ende der 3' NTR (Kolyhalov et al. ibid.) beizubehalten. Um eine interne Restriktionsstelle für das Enzym Scal zu beseitigen, wurde ferner ein sog. stiller ("silent") Nukleotidaustausch vorgenommen. Nach dem Zusammenfügen des Vollängen-Genoms mit passenden 5'- und 3' NTRen wurde die komplette HCV-Sequenz überprüft. Dabei wurde kein ungewünschter Nukleotidaustausch gefunden.

Das auf diese Weise hergestellte HCV-Genom sollte per Definition hepatotrop sein.

### (B) Synthese selektierbarer HCV-Subgenom-Konstrukte

Unter Verwendung des unter (A) beschriebenen Konsensusgenoms wurden HCV-Subgenom-Konstrukte hergestellt, die das Antibiotikumresistenzgen Neomycin-Phosphotransferase (NPT) und zwei Sequenzen von internen Ribosomenbindungsstellen (IRES) enthalten. Die hierfür angewendeten biochemischen Verfahrenstechniken sind dem Fachmann bekannt und geläufig (siehe: Sambrook, J., E.F. Fritsch, T. Maniatis, 1989, Molecularcloning: a laboratory manual, 2nd ed., Cold Spring Harbour Laboratory, Cold Spring Harbor, N.Y.; Ausubel et al. (eds.), 1994, Current Protocols in Molecular Biology, Vol. 1-3, John Wiley & Sons Inc., New York). Das Antibiotikumresistenzgen wurde unmittelbar hinter der 5' NTR insertiert, wodurch eine bicistronische RNA erhalten wurde (siehe Fig. 1 A). Ebensogut kann das Antibiotikumresistenzgen aber auch an anderer Stelle des HCV-Subgenom-Konstrukts insertiert werden, beispielsweise innerhalb der für das Polyprotein kodierenden Nukleotidsequenz, wodurch eine monocistronische RNA erhalten wird (siehe Fig. 5 A) oder in die 3' NTR (siehe Fig. 5 B). Bei den IRES-Elementen handelt es sich zum einen um eine der beiden HCV-IRES-Varianten Nukleotide 1-377 oder Nukleotide 1-389, und zum anderen um die IRES des Enzephalomyocarditis Virus, die die Translation der HCV Sequenz strangabwärts von den Genen für NS2 oder NS3 bis zu dem authentischen 3' Ende des Genoms steuert.

Die beiden genannten HCV-IRES-Varianten wurden wie folgt ermittelt:
Auf der Basis von Deletionsanalysen der 3' Grenze der HCV-IRES (Reynolds et al. 1995, *EMBO J.* 14, 6010) wurden verschiedene Abschnitte der 5' NTR mit dem NPT Gen fusioniert und anhand von Kotransfektionen mit einem das T7 RNA Polymerase Gen enthaltenden Plasmid hinsichtlich der maximalen Anzahl gebildeter Kolonien analysiert. Die besten Ergebnisse wurden mit den HCV Sequenzen von 1-377 und 1-389 erhalten. Da sich das AUG-Startkodon des HCV Polyproteins an Position 342 befindet und somit in der IRES-Sequenz enthalten ist, kommt es zu einer Fusion von 12 bzw. 16 Aminosäuren des HCV-Kapsidproteins ("Core-Proteins") mit der Neomycin Phosphotransferase (siehe Fig. 1 A).

Diese modifizierten HCV-Subgenom-Konstrukte erhielten dementsprechend die Bezeichnungen I₃₇₇/NS2-3' (oder I₃₇₇/NS3-3') und I₃₈₉/NS2-3' (oder I₃₈₉/NS3-3'). Sie sind in Fig. 1A schematisch dargestellt.

Mit in-vitro-Transkripten dieser modifizierten parentalen HCV-Subgenom-Konstrukte I₃₇₇/NS2-3' (oder I₃₇₇/NS3-3') und I₃₈₉/NS2-3' (oder I₃₈₉/NS3-3') wurden verschiedene Zellinien und Primärzellkulturen von menschlichen Hepatocyten transfiziert.

Als parallele Negativ-Kontrolle zu allen Transfektionsexperimenten wurde zu jedem modifizierten parentalen HCV-Subgenom-Konstrukt ein entsprechend modifiziertes aber defektes Subgenom konstruiert, das sich von dem parentalen dadurch unterscheidet, daß es innerhalb des Leserasters eine Deletion von 10 Aminosäuren aufweist, die das aktive Zentrum der NS5B RNA Polymerase umfaßt (Behrens et al., 1996, *EMBO J.* 15, 12; und Lohmann et al., 1997, *J. Virol.* 71, 8416).

### (C) Synthese selektierbarer HCV-Genom-Konstrukte

Ein NS2-3' Subgenomkonstrukt, das am 5' Ende mit einem Fragment des Luziferasegens und der vollständigen EMCV-IRES verbunden ist, wurde mitNcoI und SpeI restringiert und mittels präparativer Agarosegelelektrophorese gereinigt. Der so erhaltene Vektor wurde in einer 3-Faktor Ligation mit einem NcoI/NotI-HCV-Fragment, entsprechend den Nukleotidpositionen 342 bis 1968 des HCV-Genoms und mit einem NotI/SpeI-Fragment, entsprechend den Nukleotidpositionen 1968-9605 ligiert. Das entstandene Konstrukt, bei dem das vollständige HCV-Leseraster und die 3' NTR stromabwärts dem Luziferasegenfragment und der EMCV-IRES liegen, wurde danach mit PmeI und SpeI restringiert und mit dem analog restringierten I₃₈₉/NS3-3'/wt-Subgenomkonstrukt-Vektor ligiert. Dieses selektionierbare HCV-Genomkonstrukt ist in Fig. 4 dargestellt.

### (D) Herstellung von den HCV-RNA-Konstrukten entsprechenden in-vitro-Trnaskripten

Die vorstehend beschriebenen gereinigten Plasmid DNAs wurden mit ScaI linearisiert und nach Phenol/Chloroform-Extraktion und Isopropanol-Präzipitation in eine In-vitro-Trankriptionsreaktion eingesetzt unter Verwendung der folgenden Komponenten: 80 mM HEPES, pH 7.5, 12,5 mM MgCl₂, 2 mM Spermidin, 40 mM Dithiothreitol, 2 mM von jedem NTP, 1 Einheit RNasin/µl, 50 µg/ml restringierte DNA und ca. 2 Einheiten/µl T7 RNA Polymerase. Nach 2 Std. bei 37°C wurde die Hälfte der Menge an T7 Polymerase zugegeben und der Reaktionsansatz weitere 2h inkubiert. Zur Entfernung von DNA wurde die Mischung mit saurem Phenol extrahiert (U. Kedzierski, J.C. Porte, 1991, Bio Techniques 10, 210), mit Isopropanol präzipitiert, das Pellet in Wasser gelöst und mit DNase (2 Einheiten pro µg DNA) für 60 Min. bei 37°C inkubiert. Nach anschließender Extraktion mit saurem Phenol, saurem Phenol/Chloroform und Chloroform und Isopropanol- Präzipitation wurde die gelöste RNA mittel optischer Dichtemessungen quantifiziert und ihre Unversehrtheit mittels Formaldehyd-Agarose-Gelelektrophorese überprüft.

### Beispiel 2: Transfektionsexperimente mit der Hepatomazellinie Huh-7

Bei sämtlichen Transfektionsexperimenten wurde sorgfältig darauf geachtet, daß jegliche Matrizen-DNA zuvor entfernt worden war, um zu vermeiden, daß solche DNA in transfizierte Zellen integrieren und diesen unabhängig von einer HCV-Replikation eine Neomycin-Resistenz vermitteln konnte. Deshalb wurde im Anschluß an die in-vitro-Transkription ( Beispiel 1 D) die Reaktionsmischung mit 2 Einheiten DNase pro µg DNA für 60 Min. bei 37°C behandelt und mit saurem Phenol, saurem Phenol/Chloroform und Chloroform extrahiert. Vor der Verwendung fiir die Transfektion wurde die präzipitierte RNA mittels Formaldehyd Agarose Gel Elektrophorese analysiert.

Es wurden drei separate Transfektionsxperimente mit der hoch differenzierten humanen Hepatomazellinie Huh-7 (gemäß Nakabayashi et al. 1982, Cancer *Res.* **42**, 3858) durchgeführt. Dabei wurde jeweils 15 µg RNA in 8 x 10⁶ Huh-7-Zellen mit Hilfe der Elektroporation eingebracht und diese Zellen anschließend in Kulturschalen von 10 cm Durchmesser ausgesät. 24 Stunden nach der Aussaat wurde Neomycin (= G418) in einer Endkonzentration von 1 mg/ml zugegeben. Das Kulturmedium wurde zweimal pro Woche gewechselt. Nach 3 - 5 Wochen waren kleine Kolonien erkennbar, die isoliert und unter den gleichen Kulturbedingungen passagiert wurden.

Die Zellklone, die im Verlauf des ersten Experiments erhalten wurden, wurden isoliert und subpassagiert. Während dieser Prozedur starben die meisten Klone und die Endausbeute betrug nur noch 9 Klone von Zellen, die mit den parentalen HCV-Subgenom-Konstrukten transfiziert worden waren und 1 Klon (Klone 8-1) von Zellen, die mit einem defekten HCV-Genom-Konstrukt, nämlich einer defekten NS2-3' HCV-RNA transfiziert worden waren. Außer einer verkürzten Verdopplungszeit und dem gelegentlichen Auftreten von irregulär geformten Zellen wurden keine beständigen morphologischen Unterschiede zwischen diesen 9 Zellklonen und dem einen Zellklon (Klon 8-1) oder den parentalen Huh-7 Zellen gefunden.

Die Hauptkriterien für funktionierende HCV-Genomkonstrukte sind die Bildung von viraler RNA mit korrekter Größe und die Abwesenheit von (integrierter) Plasmid DNA, die eine G418-Resistenz übertragen bzw. vermitteln könnte.

Um die HCV-RNA in den Huh-7-Zellen zu bestimmen, wurde die Gesamt-RNA isoliert und mittels des gängigen Northern-Blot Verfahrens unter Verwendung einer Plusstrang-spezifischen Ribosonde (= RNA-Sonde) analysiert. Hierfür wurde von den jeweiligen Zellklonen Gesamt-RNA nach der Methode von Chomczynski und Sacchi 1987, *Anal. Biochem.* 162, 156 isoliert, und 10 µg RNA, was dem Gesamt-RNA-Gehalt von 0,5 - 1 x 10⁶ Zellen entspricht, mittels denaturierender Formaldehyd-Agarose-Gelelektrophorese aufgetrennt (Spuren 3 bis 12 der Fig. 1 B). Als Größenmarker mit authentischer Sequenz wurden gleichzeitig 10⁹ in-vitro-Transkripte (ivtr.), die zu den I₃₈₉/NS2-3'/wt oder den I₃₈₉/NS3-3'/wt Replikon-RNAs korrespondieren, mit aufgetrennt (Spur 1 bzw. Spur 2). Die aufgetrennte RNA wurde auf Nylon-Membranen transferiert und mit radioaktiv markierter Plusstrang-spezifischer RNA-Sonde, die komplementär zu dem kompletten NPT-Gen und der HCV-IRES von Nukleotid 377 bis Nukleotid 1 war, hybridisiert. Die Positionen der HCV-spezifischen RNAs (Pfeile) und der 28S rRNA sind rechts von Spur 12 angegeben, die Größen (Anzahlen der Nukleotide) der RNA-Marker sind links von Spur 1 angegeben. Die RNA Marker-Fragmente enthalten HCV-Sequenzen und hybridisieren deshalb mit der Ribosonde (= RNA-Sonde). Die Ergebnisse dieser Analyse sind in Fig. 1 B dargestellt.

Mit Ausnahme des mit dem defekten HCV-Genom-Konstrukt transfizierten Klons 8-1, lieferten alle Zellklone homogene HCV-RNAs korrekter Länge (ca. 8640 Nukleotide im Fall des NS2-3' und ca. 7970 Nukleotide im Fall des NS3-3' Replikons). Dieser Befund ist ein Indiz dafür, daß die funktionalen Replikons bzw. die funktionalen HCV-Genom-Konstrukte die G418 Resistenz übertragen. Um auszuschließen, daß die G418 Resistenz auf eine Plasmid-DNA zurückzuführen ist, die in das Genom der Huh-7 Wirtszelle integriert ist und unter der Kontrolle eines zellulären Promotors transkribiert wird, wurde von jedem Klon die DNA mittels einer NPT-Gen-spezifischen PCR untersucht. Hierbei wurde aus den selektierten Huh-7-Zellklonen die DNA mittels Verdau mit Proteinase K (40µg/ml, 1h, 37°C) in 10mMTris, pH7,5, 1mM EDTA, *0,5%* SDS und anschließender Extraktion mit Phenol, Phenol/Chloroform und Isopropanolpräzipitation isoliert. Das DNA-Präzipitat wurde in 10 mM Tris (pH 7,5) und 1 mM EDTA gelöst und 1 Stunde mit Rnase A inkubiert. Im Anschluß an eine Phenol/Chloroform Extraktion und Ethanol Präzipitation wurde 1 µg DNA, entsprechend 4 - 8 x 10⁴ Zellen, mittels PCR unter Einsatz NPT-Gen-spezifischer Primer (5'-TCAAGACCGACCTG TCCGGTGCCC-3' und 5'-CTTGAGCCTGGCGAACAGTTCGGC-3') analysiert und ein DNA-Fragment bestehend aus 379 Nukleotiden erzeugt. Die Spezifität des PCR-Produkts wurde mittels Southern Blot Verfahren nachgewiesen, wobei ein Digoxigenin-markiertes DNA Fragment eingesetzt wurde, das zu dem NPT-Gen korrespondiert. Als Positiv-Kontrollen (zum Nachweis etwa vorhandener kontaminierender Nukleinsäuren) wurde das PCR-Verfahren mit 10⁷ Plasmid Molekülen oder 1 µg DNA aus einer BHK Zellinie, die stabil mit einem Neomycin-Resistenz-Gen transfiziert war, durchgeführt, und als Negativ-Kontrolle wurde die PCR mit denselben Reagenzien aber ohne zugesetzte DNA durchgeführt.
Die Ergebnisse dieser Untersuchung sind in Fig. 1 C dargestellt. Die Spuren 1 und 2 repräsentieren die Positiv-Kontrollen, Spur 13 repräsentiert die Negativ-Kontrolle. Die Zahlenangaben links der Spur 1 bezeichnen die Größe der Nukleotid-Marker-Moleküle. Außer in Klon 7-3 (Fig. 1C, Spur 3), der von Zellen nach Transfektion mit einem NS2-3' Replikon/NS2-3'HCV-Genom-Konstrukt stammt, und in Klon 8-1 (Fig.1C, Spur12), der von Zellen nach Transfektion mit einem defekten HCV-Genom-Konstrukt stammt, war in keinem Zellklon eine NPT-DNA nachweisbar. Dieser Befund ist ein weiteres Indiz dafür, daß die G418 Resistenz der meisten Klone durch die replizierende HCV-RNA vermittelt wurde. Aber auch unabhängig von diesen Ergebnissen ist es unwahrscheinlich, daß HCV-RNAs mit korrekter Größe von integrierter Plasmid DNA erzeugt wird, denn die fiir die in-vitro-Transkription verwendeten Plasmide enthalten weder einen eukaryontischen Promotor noch ein Polyadenylierungssignal. Im Fall des Klons 7-3 ist die Resistenz deshalb höchst wahrscheinlich sowohl durch das HCV-RNA-Konstrukt bzw. die replizierende HCV-RNA als auch durch eine integrierte NPT DNA Sequenz vermittelt worden, während die Resistenz der Zellen von Klon 8-1 ausschließlich auf die integrierte Plasmid DNA zurückzuführen ist.

Um zu bestätigen, daß die G418 Resistenz von einer autonom replizierenden HCV-RNA vermittelt ist, wurde der Klon 9-13 (Fig. 1 B, Spur 11) weiteren Tests unterworfen. Klon 8-1, der integrierte Kopien des NPT-Gens trägt, wurde überall als Negativkontrolle eingesetzt. Mit dem Ziel, die Anwesenheit von NPT-DNA im Klon 9-13 rigoros auszuschließen, wurde eine PCR durchgeführt, die den Nachweis von < 1000 NPT-Gen-Kopien in ∼ 40.000 Zellen erlaubt. Das Ergebnis dieser PCR ist in Fig. 2A dargestellt. Im einzelnen wurde bei dieser PCR wie folgt verfahren:
Es wurden jeweils 10⁶ - 10² Plasmid Moleküle (I₃₇₇/NS3-3'/wt) entweder direkt (Spuren 7 - 11) oder nach Zugabe von jeweils 1 µg 9-13 DNA (Spuren 2 - 6) in dem Test eingesetzt. Die Spezifität der amplifizierten DNA Fragment wurde mittels Southern Blot unter Verwendung einer NPT-spezifischen Sonde bestimmt. Eine PCR ohne DNA-Sonde wurde als Negativ-Kontrolle durchgeführt (Spur 12).
Selbst mit dieser sensitiven Methode wurde in einem µg DNA des Zellklons 9-13 keine Plasmid DNA gefunden (Spur 1). Um die Menge an HCV Plus- und Minusstrang RNAs in diesen Zellen abzuschätzen, wurde eine Verdünnungsreihe von Gesamt-RNA mit dem Northern-Blot-Verfahren unter Verwendung einer Plus- oder Minusstrang-spezifischen radioaktiv markierten Ribosonde (= RNA-Sonde) analysiert. Hierfür wurden jeweils 8, 4 oder 2 µg Gesamt-RNA, die aus den Zellklonen 9-13 und 8-1 isoliert worden waren, parallel zu bekannten Mengen analoger in-vitro-Transkripte mit Plus- oder Minusstrang-Polarität (Kontroll-RNAs) im Northern-Blot-Vertahren analysiert und anschließend einer Hybridisierung unterworfen. Die Hybridisierung wurde mit einer Plusstrang-spezifischen Ribosonde, die das komplette NPT-Gen und die HCV-IRES abdeckte ('plusstrand', obere Bildtafel), oder mit einer Minusstrang-spezifischen RNA-Sonde, die zu der NS3-Sequenz komplementär war ('minusstrand', untere Bildtafel) durchgeführt. Die Pfeile markieren die Positionen von Replikon-RNA. Die Ergebnisse dieser Analyse sind in Fig. 2 B dargestellt.
Im Fall des Plusstrangs wurden ca. 10⁸ Kopien/µg Gesamt-RNA nachgewiesen, was 1000 - 5000 HCV-RNA-Molekülen pro Zelle entspricht, während die Menge an Minusstrang-RNA 5- bis 10-fach niedriger war. Dieses Ergebnis stimmt mit der Annahme überein, daß die Minusstrang RNA die replikative Zwischenform bzw. Zwischenkopie ist, die als Vorlage für die Synthese der Plusstrang Moleküle dient.
Da die Reaktion im wesentlichen von der viralen RNA-abhängigen RNA Polymerase katalysiert wird, sollte die Synthese der HCV-RNAs resistent gegen Dactinomycin sein, einem Antibiotikum, das selektiv die RNA-Synthese von DNA-Matrizen inhibiert, nicht jedoch die RNA-Synthese von RNA-Matrizen. Um diese Vermutung zu bestätigen, wurden Zellen mit [³H] Uridin in Anwesenheit von Dactinomycin inkubiert, die radioaktiv markierten RNAs extrahiert, mittels denaturierender Agarose-Gel-Elektrophorese aufgetrennt und mit Hilfe eines handelsüblichen Bio-Imagers unter Verwendung einer [³H]-sensitiven Bildplatte analysiert. Hierfür wurden jeweils ca. 5 x 10⁵ Zellen der Klone 9-13 und 8-1 mit 100 µ Ci [³H]Uridin für 16 Std. in Abwesenheit (-) oder Gegenwart (+) von 4 µg/ml Dactinomycin (Dact) inkubiert. Im Anschluß an diese Markierungsreaktion wurde die Gesamt-RNA präpariert und mittels Formaldehyd-Agarose-Gel-Elektrophorese analysiert. In den beiden ersten Spuren ist nur 1/10 der Gesamt-RNA dargestellt. Die radioaktiv markierte RNA wurden mit einem BAS-2500 Bio-Imager (Firma Fuji) sichtbar gemacht.
Die Ergebnisse dieser Analyse sind in Fig. 2 C dargestellt. In Übereinstimmung mit dem Inhibitor-Profil der NS5B Polymerase (Behrens et al., 1996, *EMBOJ*. 15, 12 und Lohmann et al., 1997, *J. Virol.* 71, 8416) war die Replikation der HCV RNA nicht durch Dactinomycin beeinflußt worden, während die Synthese von zellulärer RNA gehemmt worden war. Um die Identität der viralen RNA zu bestätigen, wurde eine RT-PCR zur Reklonierung der replizierten Sequenzen durchgeführt. Die Sequenzanalyse der reklonierten RNA zeigte, daß die RNA in dem Klon 9-13 HCV-spezifisch ist und mit dem transfizierten Transkript des HCV-Konstrukts I₃₇₇/NS3-3'/wt übereinstimmt.

Zur Analyse der viralen Proteine wurden die betreffenden Zellen zunächst metabolisch mit [³⁵S] Methionin/Cystein radioaktiv markiert, anschließend lysiert und danach die HCV-spezifischen Proteine mittels Immunopräzipitation aus den Zell-Lysaten isoliert. Die Ergebnisse dieser Analysen sind in Fig. 3 A dargestellt. Im einzelnen wurde dabei wie folgt verfahren: Zellen der Zellklone 9-13 (wt) und 8-1 (Δ) waren durch Behandlung für 16 Stunden mit einer dem Fachmann geläufigen und im Handel erhältlichen Protein-Markierungs-Mischung (z.B. NEN Life Science) metabolisch radioaktiv markiert worden. Mittels Immunopräzipitation (IP) unter nichtdenaturierenden Bedingungen (z.B. nach Bartenschlager et al., 1995, *J. Virol.* 69, 7519) und unter Verwendung von drei verschiedenen Antiseren (3/4, 5A, 5B, gemäß Markierung am oberen Ende der Spuren 1 bis 12) waren die HCV-spezifischen Proteine vom Zell-Lysat abgetrennt worden.. Die Immunokomplexe wurden mittels Tricine SDS-PAGE analysiert und mittels Autoradiographie sichtbar gemacht. Um authentische Größenmarker zu erhalten, wurde das homologe Replikonkonstrukt I₃₇₇/NS3-3'/wt einer transienten Expression mit dem Vaccinia Virus T7-Hybrid System in Huh-7 Zellen unterworfen. Die dabei erhaltenen Produkte waren als Größenmarker (Spuren 7 - 9) parallel zu den Zellen der Klone 9-13 und 8-1 behandelt worden. Identifizierte HCV-Proteine sind am linken Rand von Spur 1 markiert, die Molekulargewichte (in Kilodalton) sind am rechten Rand von Spur 9 angegeben. Es ist anzumerken, daß das verwendete NS3/4-spezifische Antiserum ('3/4') bevorzugt mit NS4A und NS4B reagiert, was zu einer Unterrepräsentation von NS3 führt.

Alle viralen Antigene waren eindeutig nachweisbar und ihre apparenten Molekulargewichte zeigten keine Abweichungen gegenüber denjenigen, die nach transienter Expression desselben bicistronischen HCV-RNA-Konstrukts in den ursprünglichen Huh-7 Zellen ermittelt wurden. Um die subzelluläre Verteilung der viralen Antigene zu bestimmen, wurde eine Immunofluoreszenz-Nachweisreaktion unter Einsatz von NS3- und NS5A-spezifischen Antiseren durchgeführt (z.B. nach Bartenschlager et al., 1995, *J. Virol.* 69, 7519). Hierfür wurden Zellen der Klone 9-13 (wt) und 8-1 (Δ) 24 Std. nach dem Aussäen auf Deckgläsern mit Methanol/Azeton fixiert und mit polyklonalen NS3- oder NS5A-spezifischen Antiseren inkubiert. Die gebundenen Antikörper wurden mit einem kommerziell erhältlichen FITC-konjugierten Anti-Kaninchen-Antiserum sichtbar gemacht. Zur Unterdrückung unspezifischer Fluoreszenzsignale wurden die Zellen mit dem Farbstoff 'Evans Blue' gegengefärbt.

Die Ergebnisse dieses Nachweistests sind in Fig. 3 B dargestellt. Mit beiden Antiseren war eine starke Fluoreszenz im Zytoplasma nachweisbar. Die NS5A-spezifischen Antiseren führten außerdem zu einer schwachen Zellkern-Fluoreszenz, was darauf hindeutet, daß zumindest kleine Mengen dieses Antigens auch zum Zellkern gelangen. Die generell dominierende Präsenz der viralen Antigene im Zytoplasma ist jedoch ein starkes Indiz dafür, daß die HCV-RNA Replikation im Zytoplasma stattfindet ― so wie das bei den meisten RNA-Viren der Fall ist.

Diese Ergebnisse belegen klar, daß mit dem hier beschriebenen Versuchsansatz der Aufbau eines Zellkultursystems für das HCV gelungen ist, dessen Effizienz alles bisher bekannte um Größenordnungen übersteigt und erstmalig den Nachweis viraler Nukleinsäuren und Proteine mit konventionellen und bewährten biochemischen Methoden erlaubt. Erst diese Effizienz erlaubt überhaupt detailierte Untersuchungen der HCV-Pathogenese, genetische Analysen verschiedener HCV-Funktionen und ein genaues Studium der Virus-/Wirtszellwechselwirkungen, wodurch sich neue Ansatzpunkte für die Entwicklung einer antiviralen Therapie definieren lassen.

### Beispiel 3: Transfektion von Huh-7 Zellen mit HCV-Genomkonstrukten

Huh-7 Zellen werden wie in Beispiel 2 beschrieben transfiziert und selektioniert, wobei hier jedoch selektionierbare Konstrukte verwendet werden, die das vollständige Virusgenom enthalten. Die erhaltenen Zellklone werden analog dem Beispiel 2 mittels PCR auf Abwesenheit von HCV-DNA untersucht und die produktive Replikation der HCV-RNA wird danach mittels Northern Blot, [³H]Uridinmarkierung in Anwesenheit von Dactinomycin, Nachweis der viralen Proteine bzw. Antigene vorzugsweise mit Hilfe des Western Blots, der Immunopräzipitation oder der Immunfluoreszenz nachgewiesen. Im Gegensatz zu den im Beispiel 2 beschriebenen Ansätzen lassen sich mit dem hier beschriebenen Konstrukt außerdem vollständige und sehr wahrscheinlich infektiöse Viren erhalten, was bei den dort (in Beispiel 2) beschriebenen Subgenomkonstrukten nicht der Fall ist. Diese Viren, die in der Zelle und dem Zellkulturüberstand vorhanden sind, werden beispielsweise mittels Ultrazentrifugation, Immunpräzipitation oder Fällung mit Polyethylenglykol konzentriert und alle exogenen, d.h. nicht im Viruspartikel eingebauten Nukleinsäuren werden mittels Inkubation mit Nukleasen (RNase, DNase, Mikrococusnuklease) verdaut. Auf diese Weise lassen sich alle kontaminierenden Nukleinsäuren, die nicht im schützenden Viruspartikel enthalten sind, entfernen. Die geschützte virale RNA wird nach Inaktivierung der Nukleasen, beispielsweise mittels Inkubation mit Proteinase K in einem SDS-haltigen Puffer durch Extraktion mit Phenol und Phenol/Chloroform isoliert und mittels Northern Blot oder RT-PCR unter Verwendung HCV-spezifischer Primer nachgewiesen. Auch in diesem Versuchsansatz ist die Kombination des beschriebenen HCV-Konsensusgenoms mit einem Selektionsmarker entscheidend für die effiziente Produktion von viraler RNA, viralem Protein und damit von HCV-Partikeln.

### Beispiel 4: Herstellung und Anwendung eines HCV-RNA Konstrukts, bei dem das Resistenzgen über ein Ribozym bzw. eine Erkennungsstelle für ein Ribozym mit der HCV-Subgenom-Sequenz verbunden ist.

Es wird ein HCV-RNA-Konstrukt gemäß Beispiel 1 oder Beispiel 3 hergestellt, bei dem ein Antibiotikumresistenzgen über ein Ribozym bzw. eine Erkennungsstelle für ein Ribozym mit der HCV-RNA-Sequenz verbunden ist. Solche Konstrukte sind in Fig. 7 schematisch dargestellt. Huh-7 Zellen werden wie in Beispiel 2 beschrieben mit diesem HCV-RNA-Konstrukt transfiziert. Nach der Transfektion in die Zellen erfolgt zunächst die Selektion mit dem entsprechenden Antibiotikum. In den dabei erhaltenen Zellklonen wird das einklonierte Ribozym aktiviert oder, im Fall eines Konstrukts, das eine Erkennungsstelle für ein Ribozym trägt, wird das Ribozym in die Zelle eingeschleust (z.B. mittels Transfektion eines Ribozymkonstrukts oder Infektion mit einem viralen Expressionsvektor, in den das entsprechende Ribozym eingesetzt wurde). In beiden Fällen wird durch die ribozymvermittelte Spaltung das Resistenzgen von der HCV-RNA-Sequenz abgetrennt. Das Ergebnis ist im Fall des HCV-Genom-Konstrukts ein authentisches HCV-Genom ohne Resistenzgen, das zur Bildung authentischer infektiöser Viruspartikel befähigt ist. Im Fall des HCV-Subgenom-Konstrukts entsteht ein HCV-Replikon ohne Resistenzgen.

### Beispiel 5: Kotransfektion eines HCV-RNA-Konstrukts mit einem separaten Luziferase-Transfektionskonstrukt

Es wird ein HCV-RNA-Konstrukt gemäß Beispiel 1 (A) oder Beispiel 3 oder Beispiel 4 hergestellt. Parallel dazu wird ein Transfektionskonstrukt hergestellt, welches das Luziferasegen umfaßt, wobei dieses Luziferasegen vermittels einer ersten Nukleotidsequenz, die für eine HCV-Protease- (z.B. NS3-Protease-) Spaltungsstelle kodiert, mit einer zweiten Nukleotidsequenz, die für ein anderes Protein oder einen Teil eines anderen Proteins kodiert, verbunden ist. HCV-RNA-Konstrukt und Transfektionskonstrukt werden in beliebige Wirtszellen, vorzugsweise Hepatomazellen, insbesondere Huh-7-Zellen, eingeschleust. Dies kann auf die in Beispiel 2 beschriebene Art und Weise geschehen. Das Produkt des modifizierten Luziferasegens ist ein Luziferase-Fusionsprotein, in dem die Luziferase auf Grund der Fusion mit dem Fremdanteil inaktiv ist. In transfizierten Zellen mit hoher HCV-Replikation wird das Fusionsprotein, das ja eine Schnittstelle für eine HCV-Protease enthält, gespalten und damit die aktive Form der Luziferase freigesetzt, die sich durch luminometrische Messung bestimmen läßt. Wird die Replikation des HCV-RNA-Konstrukts gehemmt, wird das Fusionsprotein nicht gespalten und keine aktive Luziferase freigesetzt. Infolgedessen ist die quantitative Bestimmung der Luziferase ein Maß für die Replikation des HCV-Subgenom-Konstrukts. Anstelle des Luziferasegens kann ebensogut ein anderes Reportergen verwendet werden, das in analoger Weise modifiziert ist, so daß seine Expression von der Virusreplikation abhängt, obwohl dieses Reportergen nicht Bestandteil des HCV-Subgenom-Konstrukts ist. Es kann auch ein zelluläres Protein, welches durch die HCV-Proteine oder Nukleinsäure inaktiviert oder aktiviert wird, als sogenannter Surrogatmarker verwendet werden. In diesem Fall ist die Expression bzw. Aktivität dieses Surrogatmarkers ein Maß für die Replikation der viralen DNA.

### Beispiel 6: Herstellung von HCV-Subgenom-Konstrukten mit integrierten Fremdgenen zur Verwendung als leberzellspezifische Genfähren für die Gentherapie

Diese rekombinanten und selektionierbaren HCV-Subgenom-Konstrukte werden in trans-komplementierende Helferzellinien transfiziert, d.h. in Zellinien, die induzierbar oder konstitutiv die fehlenden Funktionen (beispielsweise die Strukturproteine) exprimieren. Zellklone, die ein funktionelles HCV-Subgenom-Konstrukt enthalten, lassen sich durch entsprechende Selektion etablieren. Die von der Wirtszelle exprimierten Virus-Strukturproteine erlauben die Bildung von Viruspartikeln, in die die RNA der HCV-Subgenom-Konstrukte eingeschleust wird. Das Ergebnis sind also virus-ähnliche Partikel, die ein erfindungsgemäßes HCV-Subgenom-Konstrukt einschließlich des einklonierten Fremdgens enthalten und die dieses mittels Infektion auf andere Zellen übertragen können. Ein Beispiel für ein solches Konstrukt ist in Fig. 8 dargestellt. Es besteht auch die Möglichkeit, das hier beschriebene erfindungsgemäße HCV-Subgenom-Konstrukt mit integriertem Fremdgen direkt als Expressionsvektor einzusetzen. Dabei wird analog dem vorgenannten Verfahren vorgegangen, allerdings mit dem Unterschied, daß Zellinien transfiziert werden, die keine transkomplementierenden Faktoren exprimieren. In diesem Fall dient das HCV-Konstrukt also lediglich als Expressionsvektor.

### Beispiel 7: Herstellung zellkultur-adaptierter HCV-RNA-Konstrukte

### (A) Isolationsverfahren

Für die Bestimmung adaptiver Mutationen und die Herstellung zellkultur-adaptierter HCV-RNA-Konstrukte wurde wie folgt verfahren: Zellen wurden mit einem HCV-RNA-Konstrukt wie unter den Beispielen 1 und 2 beschrieben transfiziert und G418-resistente Zellklone hergestellt. Zur Bestimmung der Replikationskompetenz (darunter wird in diesem Zusammenhang die Anzahl G418-resistenter Zellklone verstanden, die pro Mikrogramm transfizierter HCV-RNA bzw. HCV-RNA-Konstrukt erhalten wird) wurde exemplarisch die Gesamt-RNA aus einem der Zellklone, genannt 9-13 (Fig. 1B, Spur 11) isoliert und die Menge der darin enthaltenen HCV-RNA mittels Northern-blot wie in Fig. 2 B beschrieben bestimmt. 10 Mikrogramm der Gesamt-RNA, die ca. 10⁹ Moleküle HCV-RNA enthielt, wurde anschließend per Elektroporation in naïve Huh-7 Zellen eingeschleust (Fig. 9). Parallel dazu wurden 10⁹ in vitro Transkripte der analogen *neo*-HCV-RNA, die mit isolierter Gesamt-RNA aus naïven Huh-7 Zellen auf eine Gesamt-RNA-Menge von 10 µg aufgefüllt worden war, in naiive Huh-7 Zellen transfiziert. Nach Selektion mit G418 wurde die Anzahl der Zellkolonien, ausgedrückt in 'colony forming units (cfu) pro Mikrogramm RNA' in den beiden Ansätzen bestimmt. Bei einer Konzentration von 500 µg/ml G418 im Selektionsmedium betrug die Zahl der Kolonien, die mit der in der ***isolierten*** Gesamt-RNA enthaltenen HCV-RNA erhalten wurde, ca. 100.000 cfu pro Mikrogramm HCV-RNA. Dagegen wurden mit derselben Menge in vitro transkribierter HCV-RNA nur 30 - 50 Kolonien erhalten. Dieses Ergebnis belegt, daß die spezifische Infektiosität der HCV-RNA, die aus den Zellklonen isoliert wurde, ca. 1.000 - 10.000-fach höher ist als die Infektiosität der analogen in vitro Transkripte. Das methodische Vorgehen ist in Fig. 9 dargestellt.

Mit Hilfe der 'long-distance RT-PCR' wurde die HCV-RNA aus der Gesamt-RNA der 9-13 Zellen amplifiziert, die PCR-Amplifikate kloniert und zahlreiche Klone sequenziert. Ein Vergleich der Sequenzen dieser reklonierten RNAs mit der Sequenz der RNA, die ursprünglich in die naiiven Huh-7 Zellen eingeschleust wurde ergab, daß die reklonierten RNAs zahlreiche Aminosäureaustausche besaßen, die über die gesamte HCV-Sequenz verteilt waren (Fig. 10). *Sfi*I-Fragmente dieser reklonierten Mutanten wurden im Austausch gegen das analoge *Sfi*I-Fragment des ursprünglichen Replikonkonstrukts in dieses eingeführt und RNAs der jeweiligen Mutanten wurden in naïve Huh-7 Zellen eingeschleust. Nach Selektion mit G418 wurde dann für jede HCV-RNA-Mutante die Zahl der gebildeten Kolonien bestimmt. Während mit der Ausgangs-RNA nur 30 - 50 Kolonien pro Mikrogramm RNA erhalten wurde war die Koloniezahl bei zwei der reklonierten Varianten deutlich höher (Fig. 10). Im Fall der HCV-RNA-Konstrukte 9-13I und 9-13C betrug die spezifische Infektiosität 100 - 1.000 cfu pro Mikrogramm RNA und beim 9-13F Replikon sogar 1.000 - 10.000 cfu pro Mikrogramm RNA. Diese Ergebnisse zeigen, daß die Aminosäureaustausche in dem analysierten NS3-5B-Bereich der Mutanten 9-13I, 9-13C und insbesondere 9-13F zu einer deutlichen Erhöhung der Replikationskompetenz führten. Demgegenüber waren alle anderen HCV-RNA-Konstrukte (9-13 A, B, G, H und K) nicht mehr replikationskompetent, enthielten also letale Mutationen.

Zwecks Beantwortung der Frage, welche der Aminosäureaustausche im 9-13F-Konstrukt zur Steigerung der Replikation führten, wurden die Austausche einzeln oder in Kombination in das Ausgangs-HCV-RNA-Konstrukt eingeführt und die entsprechenden RNAs in naïve Huh-7 Zellen eingeschleust. Das Ergebnis der Transfektionen mit diesen RNAs ist in Tabelle 1 zusammengefaßt. Daraus wird ersichtlich, daß im vorliegenden Beispiel die hohe Replikationskompetenz durch mehrere Mutationen bedingt ist. Den größten Beitrag leisten die Aminosäureaustausche in den HCV-RNA-Abschnitten NS5A und NS4B. Auch die einzelnen Austausche in der NS3-Region leisten einen Beitrag, der möglicherweise auf einem Synergismus dieser Einzelaustausche beruht.
Diese Befunde belegen, daß es durch die G418-Selektion der Zellen, die mit den *neo*-HCV-RNA-Konstrukten transfiziert wurden, zur Anreicherung solcher HCV-RNAs kam, die eine deutlich höhere Replikationskompetenz hatten. Mit dem hier beschriebenen Versuchsansatz lassen sich HCV-RNA-Konstrukte mit sehr unterschiedlicher Replikationseffizienz selektionieren. Je höher die Konzentration des Antibiotikums in dem Selektionsmedium ist, in/auf dem die HCV-RNA-Konstrukt-haltigen Zellen zwecks Selektion kultiviert werden, desto höher muß der Grad an adaptiven Mutationen und damit die Replikationseffizienz in den betreffenden HCV-RNA-Konstrukten sein, damit die Zellen auswachsen können. Werden die Selektionen mit niedrigeren Antibiotikum-Konzentrationean durchgeführt, können auch solche Zellen überleben und sich vermehren, die im Vergleich geringer adaptive Mutationen und eine weniger hohe Replikationseffizienz aufweisen.
Das bisher beschriebene HCV-RNA-Konstrukt 9-13F, das mehrere adaptive Mutationen enthielt, hatte eine erwiesenermaßen höhere Replikationseffizienz als die parentale HCV-RNA. Um HCV-RNAs mit noch höherer Replikation in Zellkultur zu erhalten, wurde die HCV-RNA, die in der Gesamt-RNA eines ausgewählten Zellklons enthalten war, mehrfach in naiven Huh-7 Zellen passagiert. Dieser ausgewählte Zellklon, genannt 5-15, wurde durch Transfektion mit dem HCV-RNA-Konstrukt I₃₈₉/NS3-3' erhalten (Fig. 1). Er entspricht weitgehend dem Zellklon 9-13, der durch Transfektion mit einem HCV-RNA-Konstrukt hergestellt wurde, das eine um 22 Nukleotide kürzere HCV-IRES besaß (I₃₇₇/NS3-3'; Fig. 1). 10 Mikrogramm Gesamt-RNA, isoliert aus dem Zellklon 5-15, wurden mittels Elektroporation in naiive Huh-7 Zellen eingeschleust und die Zellen einer Selektion mit 1 mg/ml G418 unterzogen. Aus einem der so erzeugten Zellklone wurde wiederum Gesamt-RNA isoliert, in naïve Huh-7 Zellen transfiziert und analog selektioniert. Dieser Vorgang wurde insgesamt viermal wiederholt. Nach der vierten Passage wurde aus einem Zellklon die Gesamt-RNA isoliert und die *neo*-HCV-RNA mit Hilfe der 'long-distance RT-PCR' amplifiziert. Das amplifizierte DNA-Fragment wurde mit dem Restriktionsenzym SfiI verdaut und in das SfiI-restringierte Ausgangskonstrukt I₃₈₉/NS3-3' inseriert. Insgesamt wurden über 100 DNA-Klone erhalten und zunächst mittels Restriktionsverdau analysiert. In vitro transkribierte RNA von ca. 80 dieser Klone wurde jeweils in naïve Huh-7 eingeschleust und einer Selektion mit 500mg/ml G418 unterzogen. Von den 80 untersuchten neo-HCV-RNA-Varianten erwiesen sich die allermeisten als replikationsdefekt. Bei zwei Mutanten, genannt 5.1 und 19, war die spezifische Infektiosität, ausgedrückt als 'colony forming units' pro Mikrogramm RNA, jedoch sehr deutlich erhöht (Tabelle 2). Durch mehrfache Passage der RNA in Zellkultur lassen sich offensichtlich HCV-RNAs herstellen, deren Replikationseffizienz aufgrund von Mutationen (sog. "adaptiven Mutationen) mehrere Größenordnungen höher ist als die der ursprünglich aus dem Patienten klonierten RNA.

### (B) Modifikationsverfahren

Solche nach (A) erzeugtenn und identifizierten adaptiven Mutationen können in ein wenig replikationskompetentes HCV-RNA-Konstrukt übertragen werden und führen zu einer massiven Steigerung der Replikation dieses Konstrukts. Diese Steigerung ist so hoch, daß damit nachweislich HCV-RNAs in Zellkultur zur Replikation gebracht werden können, die kein selektierbares Markergen mehr besitzen. Fig. 12 zeigt einen Vergleich der Replikationseffizienz von HCV-RNAs, die entweder der Ausgangssequenz oder den adaptierten Sequenzen 9-13F bzw. 5.1 entsprachen. Zwecks einfacher Messung wurde das *neo*-Gen entfernt und durch das Gen für die Luziferase ersetzt. Als Negativkontrolle diente wiederum ein HCV-RNA-Konstrukt, das auf Grund einer inaktivierenden Mutation der NSSB RNA-Polymerase replikationsdefekt war. Schon 24 Stunden nach der Transfektion erkennt man einen deutlichen Unterschied in der Luziferaseaktivität zwischen der defekten RNA und den 9-13F bzw. 5.1-Konstrukten während zwischen der defekten RNA (318 DN) und dem Ausgangs-RNA-Konstrukt (wt) das keine adaptiven Mutationen besaß, kaum ein Unterschied zu sehen war. Während des gesamten Beobachtungszeitraums wurde die höchste Luziferaseaktivität und damit die höchste Replikation mit der 5.1-RNA erhalten. Diese Befunde belegen nicht nur die hohe Replikationseffizienz dieser RNA, sondern zeigen auch, daß es möglich ist, mit adaptierten HCV-RNA-Konstrukten ein Zellkultursystem aufzubauen, für das die Anwesenheit eines selektierbaren Gens nicht mehr notwendig ist. Eine zusammenfassende Übersicht der Nukleotid- und Aminosäureunterschiede zwischen dem Ausgangskonstrukt und den Mutanten 9-13F, 5.1 und 19 ist in Tabelle 3 gegeben.

### Beispiel 8: Herstellung zellkultur-adaptierter HCV-RNA-Vollängengenome

In den Beispielen 1 bis 7 wurde stets eine subgenomische HCV-RNA verwendet, der die gesamte Strukturproteinregion von Core bis einschließlich p7 bzw. NS2 fehlte. Im vorliegenden Beispiel 8 wird gezeigt, daß es möglich ist, mit Hilfe der adaptierten NS3-5B-Sequenz ein HCV-Vollängengenom in Zellkultur zur Replikation zu bringen. Zu diesem Zweck wurde zunächst das SfiI-Fragment der gemäß Beispiel 7 hergestellten, hoch adaptierten HCV-RNA 5.1 in ein selektionierbares HCV-Vollängengenom transferiert (Fig. 12). Dieses HCV-Genom wurde in naïve Huh-7 Zellen transfiziert und einer Selektion mit unterschiedlichen G418-Konzentrationen unterzogen. In Abhängigkeit von der Selektionsstärke (der G418-Konzentration) wurde eine unterschiedlich große Zahl an Zellklonen erhalten (Fig. 12 B) . Im Vergleich dazu wurden mit dem unveränderten HCV-Vollängengenom, das keine-adaptiven Mutationen enthielt, keine Kolonien erhalten, ebenso mit der Negativkontrolle, die auf Grund einer inaktivierenden Mutation in der NS5B RNA-Polymerase replikationsdefekt war. Zum Nachweis dafür, daß die so entstandenen Zellklone tatsächlich ein autonom replizierendes HCV-Vollängenkonstrukt enthielten, wurde Gesamt-RNA aus mehreren Zellklonen isoliert und mittels Northern-Blot analysiert. In allen Zellklonen war die Vollängen HCV-RNA eindeutig nachweisbar (Fig. 12). Damit ist eindeutig belegt, daß es mit Hilfe der an Zellkulturen adaptierten HCV-Sequenzen möglich ist, ein HCV-Vollängengenom herzustellen, das mit hoher Effizienz und autonom in einer Zellinie repliziert, d.h. es können mit dem erfindungsgemäßen System auch adaptierte HCV-Vollängengenome hergestellt werden. Da dieser Klon darüber hinaus die vollständige HCV-Sequenz besitzt, also auch die für die Viruspartikelbildung notwendigen Strukturproteine, ist es mit diesem System möglich, große Mengen infektiöser Viruspartikel in Zellkulturen herzustellen. Zum Nachweis dieser Viren werden zellfreie Überstände von Zellen, die ein replizierendes HCV-Vollängengenom tragen, auf naive Huh-7 Zellen gegeben und die so infizierten Zellen einer Selektion mit G418 unterzogen. Jeder Zellklon, der unter diesen Bedingungen auswächst, geht auf eine infizierte Zelle zurück. Die Viren in den Zellkulturüberständen von Zellen, die ein replizierendes HCV-Vollängengenom besitzen, können aber auch mit verschiedenen im Stand der Technik bekannten Verfahren wie Ultrazentrifugation oder Mikrodialyse angereichert und gereinigt werden und dann zur Infektion naiver Zellen verwendet werden. Mit diesem Verfahren ist eindeutig gezeigt, daß mit dem erfindungsgemäßen HCV-Zellkultursystem zellkultur-adaptierte HCV-Vollängengenome hergestellt werden können, die mit hoher Effizienz in Zellen replizieren und infektiöse Viren produzieren. Diese können ebenfalls durch Infektion eines Versuchstiers, vorzugsweise dem Schimpansen, nachgewiesen werden.

### Beispiel 9: Herstellung von HCV-Vollängen-Konstrukten und HCV-Subgenom-Konstrukten mit Reportergen.

Es wird ein HCV-RNA-Konstrukt hergestellt, bei dem anstelle des Antibiotikumresistenzgens ein Reportergen eingefügt wird (Fig. 13). Dabei kann die Replikation anhand der Menge bzw. der Aktivität des Reportergens bzw. Reportergenprodukts bestimmt werden. Das Reportergen ist vorzugsweise ein Gen aus der Gruppe der Luziferasegene, dem CAT-Gen (Chloramphenicol-Acetyl-Transferase-Gen), dem lacZ-Gen (beta-Galaktosidasegen), dem GFP-Gen (green fluorescence protein Gen), dem GUS-Gen (Glukuronidasegen) oder dem SEAP-Gen (sezernierte alkalische Phosphatasegen). Diese Reportergene bzw. deren Produkte, nämlich die entsprechenden Reporterproteine, können z.B. mittels Fluoreszenz, Chemilumineszenz, colorimetrisch oder mit Hilfe immunologischer Methoden (z.B. enzyme-linked immunosorbent assay, ELISA) bestimmt werden. Das Reportergen kann entweder von einer eigenen IRES exprimiert werden oder in Form eines Fusionsproteins, das entweder als solches aktiv ist oder mittels einer proteolytisch spaltbaren Aminosäuresequenz so mit einem HCV-Protein verbunden ist, daß es von einer zellulären oder viralen (HCV-)Protease von diesem abgespalten wird.

### Beispiel 10: Herstellung von HCV-Vollängen-Konstrukten mit integrierten Fremdgenen zur Verwendung als leberzellspezifische Genfähre für die Gentherapie oder als Expressionsvektor.

Das Konstrukt (Fig. 14) wird in Zellen eingeschleust und führt dort zur Bildung von HCV-Viruspartikeln, die zur Infektion weiterer Zellen verwendet werden können. Da die Viruspartikel eine RNA mit einem Fremdgen enkapsidiert haben kann dieses in den so infizierten Zellen zur Produktion des von diesem Fremdgen kodierten Proteins benutzt werden. Zellen, die mit dem Konstrukt transfiziert wurden, exprimieren ebenfalls das Fremdgen.

### Beispiel 11: Herstellung von monocistronischen HCV-RNA-Konstrukten, bei denen das Resistenzgenprodukt als Fusionsprotein mit dem HCV-Anteil exprimiert wird.

Für bestimmte Untersuchungen ist es von Vorteil, wenn das HCV-RNA-Konstrukt kein heterologes IRES-Element besitzt. Solche Untersuchungen sind beispielsweise die Bestimmung der Interferonresistenz. Wird eine Zelle, die ein HCV-RNA-Konstrukt besitzt, mit Interferon-alpha oder -beta inkubiert, kommt es zu einer Reduktion der Replikation der HCV-RNA. Zur Aufklärung des Wirkungsmechanismus ist es notwendig, daß das HCV-RNA-Konstrukt keine heterologe IRES besitzt, da ansonsten nicht bestimmt werden kann, ob die Interferon-vermittelte Hemmung durch eine Hemmung der HCV-Replikation oder durch eine Hemmung der heterologen IRES vermittelt wird. Deshalb werden Konstrukte hergestellt, bei denen das Resistenzgen mit einem HCV-Protein fusioniert wird (Fig. 15). Entweder das Fusionsprotein ist als solches aktiv, oder das Resistenzgenprodukt wird mittels einer proteolytisch spaltbaren Aminosäuresequenz so mit einem HCV-Protein verbunden ist, daß es von einer zellulären oder viralen (HCV-)Protease von diesem abgespalten wird.

**Tabelle 1**

| Spezifische Infektiositäten (cfu/µg RNA) der HCV-RNA-Konstrukte mit adaptiven Mutationen, die bei der 9-13F Mutante gefunden und in das parentale HCV-RNA-Konstrukt I₃₈₉/NS3-3'/wt eingeführt wurden | | |
|---|---|---|
| Aminosäureaustausch¹ | HCV-Protein | cfu/µg RNA² |
| kein | | 30 - 60 |
| 1283 arg -> gly | NS3 | 200 - 250 |
| 1383 glu -> ala | NS3 | 30 - 60 |
| 1577 lys -> arg | NS3 | 30 - 60 |
| 1609 lys -> glu | NS3 | 160 - 300 |
| (1283 arg -> gly + 1383 glu -> ala + 1577 lys -> arg + 1609 lys -> glu) | NS3 | 360 - 420 |
| 1936 pro -> ser | NS4B | 500 - 1000 |
| 2163 glu -> gly | NS5A | 1000-5000 |
| 2330 lys -> glu | NS5A | 30 - 60 |
| 2442 ile -> val | NS5B | 30 - 60 |
| alle zusammen | | 5000 |

| | | |
|---|---|---|
| ¹ Aminosäureaustausch im Polyprotein des HCV-Isolats Con-1 (EMBL-Genbank No. AJ238799); die Aminosäuren sind im Dreibuchstabenkode angegeben. | | |
| ² Colony forming units (Anzahl der Zellklone) bei einer Selektion von 500µg/ml G418. | | |

**Tabelle 2**

| Spezifische Infektiositäten (cfu/µg RNA) des parentalen HCV-RNA-Konstrukts I₃₈₉/NS3-3'/wt und der Varianten 9-13C, 9-13I, 9-13F, 5.1 und 19. | |
|---|---|
| Transfizierte RNA-Variante | cfu/µg RNA¹ |
| Wildtyp | 30 - 50 |
| 9-13 C | 100 - 1.000 |
| 9-13 I | 100 - 1.000 |
| 9-13 F | 1.000 - 10.000 |
| 5.1 | 50.000 - 100.000 |
| 19 | 50.000 - 100.000 |

| | |
|---|---|
| ¹ Colony forming units (Anzahl der Zellklone) bei einer Selektion von 500µg/ml G418. | |

**Tabelle 3**

| Nukleotid- und Aminosäureunterschiede zwischen dem parentalen HCV-RNA-Konstrukt I₃₈₉/NS3-3'/wt und den Mutanten 9-13I, 9-13F, 5.1 und 19 | | | |
|---|---|---|---|
| **HCV Mutante** | **Nukleotidposition** | **Nukleotidaustausch** | **Aminosäureaustausch** |
| 9-13 I | 3685 | C > T | Pro > Leu |
| | 4933 | C > T | Thr > Met |
| | 5249 | T > C | - |
| | 8486 | C > T | - |
| | 8821 | G > A | Trp > stop |
| | 8991 | C > G | Arg > Gly |
| | 9203 | A > G | - |
| | 9313 | T > C | Phe > Ser |
| | 9346 | T > C | Val > Ala |
| 9-13 F | 3866 | C > T | - |
| | 4188 | A > G | Arg > Gly |
| | 4489 | A > C | Glu > Ala |
| | 4562 | G > A | - |
| | 4983 | T > C | - |
| | 5071 | A > G | Lys > Arg |
| | 5166 | A > G | Lys > Glu |
| | 6147 | C > T | Pro > Ser |
| | 6829 | A > G | Glu > Gly |
| | 7329 | A > G | Lys > Glu |
| | 7664 | A > G | Ile > Val |
| | 8486 | C > T | - |
| | 8991 | C > G | Arg > Gly |
| NK5.1 | 4180 | C > T | Thr > Ile |
| | 4679 | C > T | - |
| | 4682 | T > C | - |
| | 5610 | C > A | Leu > Ile |
| | 6437 | A > G | - |
| | 6666 | A > G | Asn > Asp |
| | 6842 | C > T | - |
| | 6926 | C > T | - |
| | 6930 | T > C | Ser > Pro |
| | 7320 | C > T | Pro > Ser |
| | 7389 | A > G | Lys > Glu |
| NK19 | 3946 | A > G | Glu > Gly |
| | 4078 | C > G | Ala > Gly |
| | 4180 | C > T | Thr > Ile |
| | 4682 | T > C | - |
| | 5610 | C > A | Leu > Ile |
| | 5958 | A > T | Met > Leu |
| | 6170 | T > A | - |
| | 6596 | G > A | - |
| | 6598 | C > G | Ala > Gly |
| | 6833 | C > T | - |
| | 6842 | C > T | - |
| | 6930 | T > C | Ser > Pro |
| | 7141 | A > G | Glu > Gly |
| | 7320 | C > T | Pro > Ser |
| | 7389 | A > G | Lys > Glu |
| | 7735 | G > A | Ser > Asn |

Angegeben sind die Unterschiede der Nukleotid- und Aminosäuresequenzen zwischen der Ausgangs-HCV-RNA-Sequenz Con 1 (EMBL-Genbank No. AJ238799) und denen der zellkulturadaptierten HCV-RNAs. Die Zahlen beziehen sich auf die Nukleotid- und Aminosäurepositionen des HCV-Isolats Con1.

## Patentansprüche

1. Hepatitis-C-Virus (HCV)-RNA-Konstrukt mit der Fähigkeit zur Replikation in eukaryontischen Zellen, **dadurch gekennzeichnet,**
**daß** es eine Nukleotidsequenz gemäß einem der Sequenzprotokolle SEQ ID NO: 1 bis SEQ ID NO: 11 umfaßt, welche zumindest für die HCV-spezifischen RNA-Abschnitte 5' NTR, NS3, NS4A, NS4B, NS5A, NSSB und 3' NTR und zusätzlich ein selektierbares Markergen (Selektionsgen) kodieren, wobei das selektierbare Markergen das Neomycinphosphotransferasegen ist und wobei anstelle des Neomycinphosphotransferasegens ein anderes Antibiotikaresistenzgen oder sonstiges Resistenzgen enthalten sein kann.

2. HCV-RNA-Konstrukt nach Anspruch 1, **dadurch gekennzeichnet,**
**daß** es anstelle des Markergens oder zusätzlich zu dem Markergen ein integriertes Reportergen aufweist.

3. HCV-RNA-Konstrukt nach Anspruch 2, **dadurch gekennzeichnet,**
**daß** das Reportergen ausgewählt ist aus der Gruppe bestehend aus den Luziferasegenen, dem CAT-Gen (Chloramphenicol-Acetyl-Transferase-Gen), dem lacZ-Gen (beta-Galaktosidase-Gen), den GFP-Genen (green-fluorescence-protein-Genes), dem GUS-Gen (Glukuronidasegen) und dem SEAP-Gen (Sezernierte-Alkalische-Phosphatase-Gen).

4. HCV-RNA-Konstrukt nach einem der Ansprüche 1 bis 3, **dadurch gekennzeichnet, daß** dessen Replikation die Expression eines (zellulären) Surrogatmarkergens beeinflußt.

5. HCV-RNA-Konstrukt nach einem der Ansprüche 1 bis 4, **dadurch gekennzeichnet, daß** das Reportergen und das selektierbare Markergen derart räumlich in dem Konstrukt angeordnet sind, daß sie gemeinsam ein Fusionsprotein exprimieren.

6. HCV-RNA-Konstrukt nach einem der Ansprüche 1 bis 5, **dadurch gekennzeichnet, daß** es ein integriertes Fremdgen aufweist und dazu geeignet ist, dieses Fremdgen in eine Zielzelle einzuschleusen, die zur Expression dieses Fremdgens geeignet ist.

7. HCV-RNA-Konstrukt nach einem der Ansprüche 1 bis 6, **dadurch gekennzeichnet, daß** es Nukleotid- und/oder Aminosäure-Mutationen aufweist, zellkultur-adaptiert ist, mit hoher Effizienz repliziert und dadurch erhältlich ist, daß man ein Zellkultursystem gemäß Anspruch 11, bei dem das eingeschleuste HCV-spezifische Genmaterial ein HCV-RNA-Konstrukt mit Selektionsgen nach einem der Ansprüche 1 bis 6 ist, auf/in dem dem Selektionsgen entsprechenden Selektionsmedium kultiviert, daß man die gewachsenen Zellklone erntet, und daß man aus diesen Zellklonen das HCV-RNA-Konstrukt oder Teile davon isoliert.

8. HCV-RNA-Konstrukt nach Anspruch 7, **dadurch gekennzeichnet, daß** man das aus den Zellklonen isolierte HCV-RNA-Konstrukt oder Teile davon wenigstens einmal erneut passagiert, nämlich in Zellen des Zellkultursystems gemäß Anspruch 11 einschleust und diese Zellen auf/in dem dem Selektionsgen entsprechenden Selektionsmedium kultiviert, daß man die gewachsenen Zellklone erntet, und daß man aus diesen Zellklonen das HCV-RNA-Konstrukt oder Teile davon isoliert.

9. HCV-RNA-Konstrukt gemäß einem der Ansprüche 1 bis 8, **dadurch gekennzeichnet,**
**daß** es einen oder mehrere der nachfolgend aufgeführten Aminosäureaustausche aufweist, nämlich 1283 arg -> gly und/oder 1383 glu -> ala und/oder 1577 lys -> arg und/oder 1609 lys -> glu und/oder 1936 pro -> ser und/oder 2163 glu -> gly und/oder 2330 lys -> glu und/oder 2442 ile -> val aufweist, zellkultur-adaptiert ist und mit hoher Effizienz repliziert.

10. HCV-RNA-Konstrukt gemäß einem der Ansprüche 1 bis 9, **dadurch gekennzeichnet,**
**daß** es einen oder mehrere der in Tabelle 3 aufgeführten Nukleotid- und/oder Aminosäureaustausche aufweist, wobei Tabelle 3 Bestandteil dieses Anspruchs ist.

11. HCV Zellkultursystem, das im wesentlichen eukaryontische Zellen umfaßt, die eingeschleustes HCV-spezifisches Genmaterial enthalten, **dadurch gekennzeichnet, daß** die eukaryontischen Zellen humane Hepatomazellen sind und daß das eingeschleuste HCV-spezifische Genmaterial ein HCV-RNA-Konstrukt gemäß einem der Ansprüche 1 bis 10 ist.

12. Zellkultursystem nach Anspruch 11, **dadurch gekennzeichnet,**
**daß** die das HCV-RNA-Konstrukt enthaltenden Zellen bei der DSMZ, Braunschweig, BRD, unter der Hinterlegungsnummer DSM ACC2394 (Laborbezeichnung HuBl 9-13) hinterlegt sind.

13. Verwendung eines Zellkultursystems nach einem der Ansprüche 11 oder 12 und/oder eines HCV-RNA-Konstrukts nach einem der Ansprüche 1 bis 10 zur Herstellung und/oder Evaluierung von Therapeutika und/oder Diagnostika für HCV-Infektionen.

14. Verwendung eines Zellkultursystems nach einem der Ansprüche 11 oder 12 und/oder eines HCV-RNA-Konstrukts nach einem der Ansprüche 1 bis 10 zur Herstellung eines Impfstores gegen HCV-Infektionen.

15. Verwendung eines HCV-RNA-Konstrukts nach einem der Ansprüche 1 bis 10 zur Herstellung einer leberzellspezifischen Genfähre für die Gentherapie.

16. Verwendung eines HCV-RNA-Konstrukts nach einem der Ansprüche 1 bis 6 zur Gewinnung von zellkultur-adaptierten Mutanten dieses HCV-RNA-Konstrukts, wobei die Mutanten gegenüber dem HCV-RNA-Konstrukt eine erhöhte Replikationseffizienz aufweisen, **dadurch gekennzeichnet,**
**daß** man ein Zellkultursystem gemäß Anspruch 11 oder 12, das HCV-RNA-Konstrukte gemäß einem der Ansprüche 1 bis 6 enthält, auf/in dem dem Selektionsgen entsprechenden Selektionsmedium kultiviert, daß man die gewachsenen Zellklone erntet, und daß man aus diesen Zellklonen die HCV-RNA-Konstrukte oder Teile davon isoliert.

17. Verwendung eines HCV-RNA-Konstrukts nach Anspruch 16, **dadurch gekennzeichnet,**
**daß** man die isolierten HCV-RNA-Konstrukte wenigstens einmal erneut passagiert, nämlich in Zellen eines Zellkultursystems nach Anspruch 11 oder 12 einschleust und diese auf/in dem dem Selektionsgen entsprechenden Selektionsmedium kultiviert, die gewachsenen Zellklone erntet und aus diesen Zellklonen die HCV-RNA-Konstrukte oder Teile davon isoliert.

18. Verwendung eines HCV-RNA-Konstrukts nach einem der Ansprüche 1 bis 6 zur Herstellung von Mutanten eines HCV-Vollängengenoms oder eines HCV-Teilgenoms oder eines beliebigen HCV- Konstrukts mit im Vergleich zu dem ursprünglichen HCV-Vollängengenom oder -Teilgenom oder HCV-RNA-Konstrukt erhöhter Replikationseffizienz, **dadurch gekennzeichnet,**
- **daß** man gemäß Anspruch 16 oder Anspruch 17 eine zellkultur-adaptierte Mutante des HCV-RNA-Konstrukts herstellt,
- **daß** man die Nukleotid- und Aminosäuresequenz dieser Mutante bestimmt und durch Vergleich mit der Nukleotid- und Aminosäuresequenz des ursprünglichen HCV-RNA-Konstrukts die Art, Anzahl und Positionen der Nukleotid- und Aminosäuremutationen bestimmt,
- und **daß** man diese Mutationen entweder durch gezielte Mutagenese oder durch Austausch von Sequenzabschnitten, die die betreffenden Mutationen enthalten, in ein (isoliertes) HCV-Vollängengenom oder ein HCV-Teilgenom oder ein beliebiges HCV-RNA-Konstrukt einführt.

19. Verwendung eines HCV-RNA-Konstrukts nach einem der Ansprüche 1 bis 10 zur Erzeugung von Hepatitis C Viruspartikeln oder virus-ähnlichen Partikeln.

## Claims

1. Hepatitis-C-virus (HCV) RNA construct with the ability to replicate in eukaryotic cells, **characterized in**
**that** it comprises a nucleotide sequence in accordance with one of the sequences listed as SEQ ID NO: 1 to SEQ ID NO: 11, which codes at least for the HCV specific RNA-segments 5'NTR, NS3, NS4A, NS4B, NS5A, NS5B and 3'NTR and additionally for a marker gene for selection (selection gene), whereby the marker gene for selection is the neomycin phosphotransferase gene and whereby another antibiotic resistance gene or other selection gene may be contained in place of the neomycin phosphotransferase gene.

2. HCV RNA construct according to claim 1, **characterized in**
**that** it comprises instead of the marker gene or in addition to the marker gene an integrated reporter gene.

3. HCV RNA construct according to claim 2, **characterized in**
**that** the reporter gene is a gene from the group consisting of the luciferase genes, the CAT-gene (chloramphenicol acetyl transferase gene), the lacZ gene (beta galactosidase gene), the GFP genes (green fluorescence protein genes), the GUS gene (glucuronidase gene) and the SEAP gene (secreted alkaline phosphatase gene).

4. HCV RNA construct according to claims 1 to 3, **characterized in**
**that** its replication influences the expression of a (cellular) surrogate marker gene.

5. HCV RNA construct according to claim 1 to 4, **characterized in**
**that** the reporter gene and the marker gene for selection are spatially arranged in the construct in such a way that they are expressed together as a fusion protein.

6. HCV RNA construct according to claim 1 to 5, **characterized in**
**that** it comprises an integrated foreign gene and can be used to introduce this foreign gene into a target cell which is suited for expressing this foreign gene.

7. HCV RNA construct according to claim 1 to 6, **characterized in that** it comprises nucleotide and/or amino acid mutations, that it is cell culture-adapted, that it replicates with high efficiency and that it is obtained by using a cell culture system according to claim 11, in which the transferred HCV specific gene material is an HCV RNA construct with a selection gene according to one of the claims 1 to 6, by culturing on/in the selection medium that is appropriate for the particular selection gene, by harvesting the grown cell clones, and by isolating the HCV RNA construct or parts thereof from these cell clones.

8. HCV RNA construct according to claim 7, whereby the isolated HCV-RNA-construct or parts thereof are subjected to at least one further passage, i.e. are introduced into cells of a cell culture system according to claim 11, these cells are cultivated on/in the selection medium that is appropriate for the particular selection gene, the grown cell clones are harvested and the HCV RNA construct or parts thereof are isolated from these cell clones.

9. HCV RNA construct according to one of the claims 1 to 8, **characterized in**
**that** it comprises one or several of the following specified amino acid exchanges, namely 1283 arg -> gly and/or 1383 glu -> ala and/or 1577 lys -> arg and/ or 1609 lys - > glu and/or 1936 pro - > ser and/or 2163 glu - > gly and/or 2330 lys - > glu and/or 2442 ile - > val , that it is cell culture-adapted and that it replicates with high efficiency.

10. HCV RNA construct according to claim 1 to 9, **characterized in**
**that** it comprises one or several of the nucleotide and/or amino acid exchanges specified in table 3, whereby table 3 is constituent of this claim.

11. HCV cell culture system, composed essentially of eukaryotic cells, which contain transferred HCV specific genetic material, **characterized in that** the eukaryotic cells are human hepatoma cells and that the transferred HCV specific genetic material is an HCV RNA construct in accordance with one of the claims 1 to 10.

12. Cell culture system according to claim 11, **characterized in**
**that** the cells containing the HCV RNA construct are deposited at the DSMZ, Braunschweig, FRG, under the deposit number DSM ACC2394 (laboratory name HuBl 9-13).

13. A use of a cell culture system according to claim 11 or 12 and/or a HCV RNA construct according to one of the claims 1 to 10 for the production and/or evaluation of therapeutics and/or diagnostics for HCV infections.

14. A use of a cell culture system according to claim 11 or 12 and/or a HCV RNA construct according to one of the claims 1 to 10 for the production of a vaccine against HCV infections.

15. A use of an HCV RNA construct according to one of the claims 1 to 10 for the production of a liver-cell-specific vector in gene therapy.

16. A use of an HCV RNA construct according to one of the claims 1 to 6 for the production of cell culture-adapted mutants of this HCV RNA construct, whereby the mutants replicate with increased efficiency in comparison with the HCV RNA construct, **characterized in that** one cultivates a cell culture system in accordance with claim 11 or 12, which contains HCV RNA constructs in accordance with one of the claims 1 to 6 on/in the selection medium that is appropriate for the particular selection gene, that one harvests the grown cell clones, and that one isolates the HCV RNA constructs or parts thereof them from theses cell clones.

17. A use of an HCV RNA construct according to claim 16, **characterized in**
**that** one performs at least one further passage with the isolated HCV RNA constructs, i.e. one introduces the isolated HCV RNA constructs into cells of a cell culture system according to claim 11 or 12 and cultivates these cells on/in the selection medium that is appropriate for the particular marker gene, one harvests grown cell clones and isolates the HCV RNA constructs or parts thereof from these cell clones.

18. Use of an HCV RNA construct according to one of the claims 1 to 6 for the production of mutants of an HCV full length genome or an HCV partial genome or any HCV construct having an increased replication efficiency in comparison with the original HCV full length genome or partial genome or HCV RNA construct, **characterized in**
- **that** one creates a cell culture-adapted mutant of the HCV RNA construct in accordance with claim 16 or claim 17,
- **that** one analyzes the nucleotide and amino acid sequence of this mutant and by comparison with the nucleotide and amino acid sequence of the original HCV RNA construct determines the type, number and positions of the nucleotide and amino acid mutations in which the original HCV RNA construct differs from this mutant,
- and **that** one introduces these mutations found in the mutant either by direct mutagenesis or by exchange of fragments of the HCV RNA construct, which contain the mutations concerned into an (isolated) HCV full length genome or an HCV partial genome or any HCV RNA construct.

19. A use of an HCV RNA construct according to one of the claims 1 to 10 for the production of hepatitis C virus particles or virus-like particles.

## Revendications

1. Construction d'ARN du virus de l'hépatite C (VHC) avec la capacité de réplication dans des cellules eucaryotes, **caractérisée en ce qu'**elle comprend une séquence de nucléotides selon l'un des protocoles de la séquence SEQ ID N° : 1 à SEQ ID N° : 11, codant pour au moins les sections d'ARN spécifiques du VHC 5' NTR, NS3, NS4A, NS4B, NS5A, NS5B et 3'NTR et en outre, un gène marqueur sélectionnable (gène de sélection), le gène marqueur sélectionnable étant le gène de la néomycine phosphotransférase et un autre gène résistant aux antibiotiques ou un autre gène résistant pouvant être contenu à la place du gène de néomycine-phospho-transférase.

2. Construction d'ARN du VHC selon la revendication 1, **caractérisée en ce qu'**elle présente, à la place du gène marqueur ou en complément du gène marqueur, un gène rapporteur intégré.

3. Construction d'ARN du VHC selon la revendication 2, **caractérisée en ce que** le gène reporter est sélectionné dans le groupe constitué des gènes de la luciférase, du gène CAT (gène de la chloramphénicol acétyltransférase), du gène lacZ (gène bêta-galactosidase), des gènes GFP (gène de la protéine vert fluorescent), du gène GUS (gène de la glucuronidase) et du gène SEAP (gène de la phosphatase alcaline sécrétoire)

4. Construction d'ARN du VHC selon l'une des revendications 1 à 3, **caractérisée en ce que** sa réplication influe sur l'expression d'un gène marqueur substitut (cellulaire).

5. Construction d'ARN du VHC selon l'une des revendications 1 à 4, **caractérisée en ce que** le gène reporter et le gène marqueur sélectionnable sont agencés de telle façon dans l'espace de la construction qu'ils expriment ensemble une protéine de fusion.

6. Construction d'ARN du VHC selon l'une des revendications 1 à 5, **caractérisée en ce qu'**elle présente un gène étranger intégré et est apte à introduire ce gène étranger dans une cellule cible qui est apte à exprimer ce gène étranger.

7. Construction d'ARN du VHC selon l'une des revendications 1 à 6, **caractérisée en ce qu'**elle présente des mutations de nucléotide et / ou d'acides aminés, elle est adaptée à la culture cellulaire, elle se réplique avec une efficacité élevée et elle peut être obtenue en cultivant un système de culture cellulaire selon la revendication 11, dans lequel le matériel génétique spécifique au VHC introduit est une construction d'ARN du VHC avec un gène de sélection selon l'une des revendications 1 à 6, sur / dans le milieu de sélection correspondant au gène de sélection, en récoltant les clones cellulaires cultivés et en isolant de ces clones cellulaires la construction d'ARN du VHC ou des parties de celle-ci.

8. Construction d'ARN du VHC selon la revendication 7, **caractérisée en ce que** l'on effectue au moins une fois de nouveau un passage de la construction d'ARN du VHC isolée à partir des clones cellulaires ou des parties de celle-ci, à savoir que l'on introduit ladite construction d'ARN du VHC dans des cellules du système de culture cellulaire selon la revendication 11 et que l'on cultive ces cellules sur / dans le milieu de sélection correspondant au gène de sélection, que l'on récolte les clones cellulaires cultivés et que l'on isole de ces clones cellulaires la construction d'ARN du VHC ou des parties de celle-ci.

9. Construction d'ARN du VHC selon l'une des revendications 1 à 8, **caractérisée en ce qu**'elle présente un ou plusieurs échanges des acides aminés suivants, à savoir 1283 arg-> gly et / ou 1383 glu-> ala et / ou 1577 lys-> arg et / ou 1609 lys-> glu et / ou 1936 pro-> ser et / ou 2163 glu-> gly et / ou 2330 lys-> glu et / ou 2442 ile-> val, qu'elle est adaptée à la culture cellulaire et elle se réplique avec une efficacité importante.

10. Construction d'ARN du VHC selon l'une des revendications 1 à 9, **caractérisée en ce qu'**elle présente un ou plusieurs échanges de nucléotides et / ou d'acides aminés figurant dans le tableau 3, le tableau 3 faisant partie intégrante de cette revendication.

11. Système de culture cellulaire du VHC qui comprend essentiellement des cellules eucaryotes contenant un matériel génétique spécifique au VHC introduit, **caractérisé en ce que** les cellules eucaryotes sont des cellules d'hépatome humain et **en ce que** le matériel génétique spécifique du VHC introduit est une construction d'ARN du VHC selon l'une des revendications 1 à 10.

12. Système de culture cellulaire selon la revendication 11, **caractérisé en ce que** les cellules contenant la construction d'ARN du VHC sont enregistrées auprès du DSMZ, Braunschweig, RFA, sous le numéro d'enregistrement DSM ACC2394 (code laboratoire HuBI 9-13).

13. Utilisation d'un système de culture cellulaire selon l'une des revendications 11 ou 12 et / ou d'une construction d'ARN du VHC selon l'une des revendications 1 à 10 pour la production et / ou l'évaluation de produits thérapeutiques et / ou diagnostiques pour les infections au VHC.

14. Utilisation d'un système de culture cellulaire selon l'une des revendications 11 ou 12 et / ou d'une construction d'ARN du VHC selon l'une des revendications 1 à 10 pour la production d'un vaccin contre les infections au VHC.

15. Utilisation d'une construction d'ARN du VHC selon l'une des revendications 1 à 10 pour la production d'un vecteur génétique spécifique des cellules hépatiques pour la thérapie génique.

16. Utilisation d'une construction d'ARN du VHC selon l'une des revendications 1 à 6 pour obtenir des mutants de cette construction d'ARN du VHC adaptés à la culture cellulaire, les mutants présentant par rapport à la construction d'ARN du VHC une efficacité de réplication accrue, **caractérisée en ce que** l'on cultive un système de culture cellulaire selon la revendication 11 ou 12 contenant la construction ARN du VHC selon l'une des revendications 1 à 6, sur / dans le milieu de sélection correspondant au gène de sélection, que l'on récolte les clones cellulaires cultivés et que l'on isole de ces clones cellulaires des constructions d'ARN du VHC ou des parties de celles-ci.

17. Utilisation d'une construction d'ARN du VHC selon la revendication 16, **caractérisée en ce que** l'on effectue au moins une fois de nouveau un passage de la construction d'ARN du VHC isolée, à savoir que l'on introduit ladite construction d'ARN du VHC dans les cellules d'un système de culture cellulaire selon la revendication 11 ou 12 et que l'on cultive celles-ci sur / dans le milieu de sélection correspondant au gène de sélection, que l'on récolte les clones cellulaires cultivés et que l'on isole de ces clones cellulaires la construction d'ARN du VHC ou des parties de celle-ci.

18. Utilisation d'une construction d'ARN du VHC selon l'une des revendications 1 à 6, pour la production de mutants d'un génome entier du VHC ou d'un génome partiel du VHC ou d'une construction quelconque du VHC avec, par rapport au génome entier du VHC ou au génome partiel ou à la construction d'ARN du VHC d'origine, une efficacité de réplication accrue, **caractérisée en ce que**
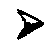 l'on produit selon la revendication 16 ou 17 un mutant adapté à la culture cellulaire de la construction d'ARN du VHC,
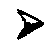 que l'on détermine la séquence de nucléotides et d'acides aminés de ce mutant et que l'on détermine, par comparaison avec la séquence de nucléotides et d'acides aminés de la construction d'ARN du VHC d'origine, la nature, le nombre et les positions des mutations des nucléotides et des acides aminés,
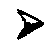 et que l'on introduit ces mutations soit par une mutagenèse ciblée, soit par l'échange de sections de séquences contenant les mutations concernées, dans un génome entier du VHC (isolé) ou un génome partiel du VHC ou une construction d'ARN du VHC quelconque.

19. Utilisation d'une construction d'ARN du VHC selon l'une des revendications 1 à 10 pour la production de particules virales de l'hépatite C ou de particules de type viral.
